# EUROPÄISCHE PATENTSCHRIFT

(11) **EP 0 351 352 B1**
(45) Veröffentlichungstag und Bekanntmachung des Hinweises auf die Patenterteilung: **08.03.1995**
(21) Anmeldenummer: 89730164.4
(22) Anmeldetag: 14.07.1989
(51) Int. Cl.: C07D 457/12, A61K 31/48

(54) **2-Substituierte Ergoline, ihre Herstellung und Verwendung**
2-Substituted ergolines, their preparation and use
Ergoline substituées en 2, leur préparation et utilisation

(30) Priorität: 15.07.1988 DE 3824661; 23.05.1989 DE 3917268
(43) Veröffentlichungstag der Anmeldung: 17.01.1990
(73) Patentinhaber: SCHERING AKTIENGESELLSCHAFT, 13353 Berlin (DE)
(72) Erfinder: Brumby, Thomas, Dr., D-1000 Berlin 62 (DE); Sauer, Gerhard, Dr., D-1000 Berlin 65 (DE); Heindl, Josef, Dr., D-1000 Berlin 38 (DE); Turner, Jonathan, Dr., D-1000 Berlin 28 (DE); Kühne, Gerhard, D-1000 Berlin 27 (DE); Wachtel, Helmut, Dr., D-1000 Berlin 19 (DE)

(56) Entgegenhaltungen:
- EP-A- 0 021 206
- EP-A- 0 160 842
- EP-A- 0 185 392
- EP-A- 0 217 735
- EP-A- 0 250 357
- EP-A- 0 286 575
- DE-A- 3 500 251

## Beschreibung

Die Erfindung betrifft 2-substituierte Ergolinylharnstoffderivate, ihre Herstellung und Verwendung als Zwischenprodukte sowie in Arzneimitteln.

2-substituierte Ergolinylharnstoffderivate sind aus der EP-A-160 842 und EP-A-250 357 bekannt, wobei auf Grund ihrer apomorphin-antagonistischen Wirksamkeit und/oder ihrer α₂-rezeptor-blockierenden Wirkung, die beschriebenen 2-substituierten Derivate insbesondere als Neuroleptika geeignet sind.

Aus DE-A-3 500 251 und aus EP-A-21206 sind ähnlich substituierte Ergolinderivate bekannt, die dopaminerge Aktivität seigen.

Überraschenderweise wurde nun gefunden, daß bei Einführung eines längerkettigen Kohlenwasserstoffrestes in 6-Stellung an 2-substituierten Ergolinylharnstoffderivaten, die Wirkung vom Dopaminantagonismus zum Dopaminagonismus verschoben wird, und gleichzeitig die metabolische Stabilität der Verbindungen erhalten bleibt oder verbessert wird.

Die Erfindung betrifft Verbindungen der allgemeinen Formel I 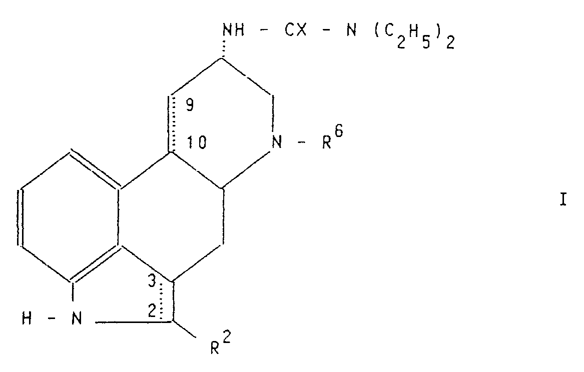
worin 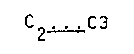
und 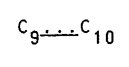
eine Einfach- oder Doppelbindung,
- X: Sauerstoff oder Schwefel
- R²: C₁₋₁₀-Alkyl, gegebenenfalls mit Halogen substituiertes C₂₋₁₀-Alkenyl, CH₂YR³, CR¹²(OR⁴)R⁵, CH₂-CHR⁹-COR¹⁰ oder COR¹²
worin Y Sauerstoff oder Schwefel, R³ Wasserstoff, C₁₋₄-Alkyl, Phenyl, C₂₋₅-Acyl oder Phenyl-C₁₋₄-Alkyl, R⁴ Wasserstoff oder C₂₋₅-Acyl, R⁵ C₁₋₉-Alkyl, R⁹ COCH₃ oder COO-C₁₋₄-Alkyl, R¹⁰ C₁₋₄-Alkyl oder O-C₁₋₄-Alkyl, R¹² Wasserstoff oder C₁₋₉-Alkyl bedeuten und
- R⁶: C₂₋₁₀-Alkyl, C₃₋₁₀-Alkenyl oder C₃₋₇-Cycloalkyl-C₁₋₃-alkyl sind sowie
deren Säureadditionssalze, Isomeren und Isomerengemische.

Stellt 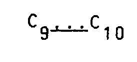
eine Einfachbindung dar, so steht das Wasserstoffatom in 10-Stellung α-ständig, stellt 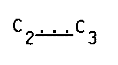
eine Einfachbindung dar, so steht das Wasserstoffatom in 3-Stellung β-ständig. Die Verbindungen der Formel I können als E- oder Z-Isomere oder, falls ein chirales Zentrum im Rest R² vorhanden ist, als Diastereomere und als Gemische derselben auftreten.

Unter Alkyl ist jeweils ein geradkettiger oder verzweigter Alkylrest zu verstehen, wie beispielsweise Methyl, Ethyl, Propyl, Isopropyl, Butyl, Isobutyl, sek. Butyl, tert. Butyl, Pentyl, Hexyl, Heptyl, Octyl, Nonyl, 1,2-Dimethylheptyl, Decyl, 2,2-Dimethylpropyl,2-Methylbutyl, 3-Methylbutyl u.a.

Alkenyl bedeutet jeweils beispielsweise Vinyl, 1-Propenyl, 2-Propenyl, 3-Methyl-2-propenyl, 1-Butenyl, Methallyl.

Der Alkenylrest R² kann ein- oder zweifach mit Halogen wie Fluor, Chlor, Brom oder Jod substituiert sein, wobei Fluor und Chlor bevorzugt sind.

Als Acylgruppen sind jeweils aliphatische Carbonsäuren geeignet wie beispielsweise Essigsäure, Propionsäure, Buttersäure, Capronsäure und Trimethylessigsäure u.a.

Bedeutet R⁶ eine Cycloalkyl-alkyl-Gruppe, so ist beispielsweise Cyclopropylmethyl, Cyclopropylethyl, Cyclobutylmethyl, Cyclobutylethyl, Cyclopentylmethyl, Cyclohexylmethyl, Cycloheptylmethyl u.a. gemeint.

Bevorzugt sind für den Rest R⁶ Alkyl-, Alkenyl und Cycloalkyl-alkyl- mit bis zu 4-C-Atomen. Die Reste R⁵ und R¹² sollen zusammen nicht 10 vorzugsweise nicht 6 Kohlenstoffatome überschreiten.

Die physiologisch verträglichen Säureadditionssalze leiten sich von den bekannten anorganischen und organischen Säuren ab, wie z.B. Salzsäure, Schwefelsäure, Bromwasserstoffsäure, Zitronensäure, Maleinsäure, Fumarsäure oder Weinsäure.

Die Verbindungen der allgemeinen Formel I und ihre pharmazeutisch akzeptablen Säureadditionssalze weisen pharmakologische Wirkungen, insbesondere zentral dopaminerge Wirksamkeit auf, und sind daher als Arzneimittel verwendbar.

Die dopaminagonistische Wirkung wurde mit Hilfe der von Horowski beschriebenen Methode der automatischen Registrierung von Stereotypien an Ratten bestimmt (Arzneim. Forsch. 12, 2281-2286, 1978): Unmittelbar nach intraperitonealer Prüfsubstanz- bzw. Vehikelverabreichung werden männliche Wistar-Ratten (90-120 g) einzeln in Zwangskäfige aus Acrylglas gesetzt. Über ein vor dem Kopf der Tiere angebrachtes elektrodynamisches Aufnahmesystem wird die Zahl der Kontakte an einem stählernen Becher mit einem zentralen Metallstab als Folge der stereotypen Kau-, Leck- und Nagebewegungen während 60 Minuten registriert. Die Mittelwerte ± S.E.M. der Anzahl der Kontakte während 60 Minuten für die verschiedenen Behandlungsgruppen, die mit jeweils 12 Tieren besetzt sind, werden berechnet und die Signifikanz der Unterschiede zwischen den Mittelwerten der verschiedenen Prüfsubstanzdosen im Vergleich zur Vehikel-behandelten Kontrollgruppe mit Hilfe der einfachen Varianzanalyse in Verbindung mit dem Dunnett-Test ermittelt.

Da sich die erfindungsgemäßen Verbindungen durch dopaminagonistische Wirkung auszeichnen, eignen sie sich insbesondere zur Behandlung des Morbus Parkinson.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Ole, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Die Herstellung der erfindungsgemäßen Verbindungen der Formel I kann nach an sich bekannten Methoden durchgeführt werden.

Beispielsweise gelangt man zu Verbindungen der Formel I, in dem man
a) eine Verbindung der Formel II 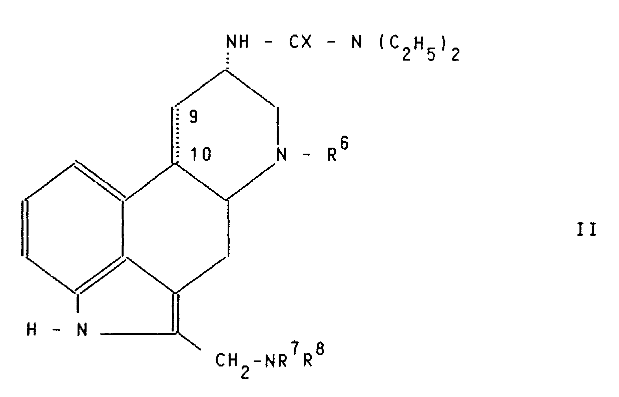 worin R⁶, X und 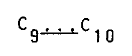 die obige Bedeutung haben und R⁷ und R⁸ jeweils C₁₋₆-Alkyl oder gemeinsam mit dem Stickstoffatom einen 5-6 gliedrigen gesättigten Heterocyclus, der gegebenenfalls durch ein Sauerstoffatom unterbrochen sein kann, bedeuten, zum Aldehyd der Formel Ia oxidiert 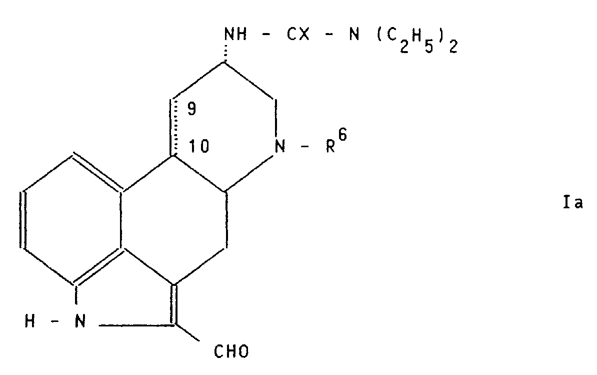 worin R⁶, X und 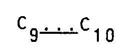 die obige Bedeutung besitzen,
b) das quartäre Salz der Formel IIa 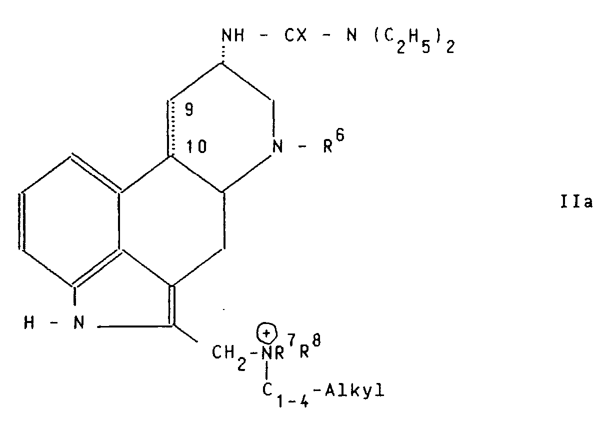 worin R⁶, R⁷, R⁸, X und 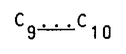 die obige Bedeutung haben, reduziert zu einer Verbindung der Formel I, worin R² Methyl ist oder mit einem nucleophilen Anion umsetzt und gewünschtenfalls in einer so erhaltenen Verbindung der Formel I, worin R² für CH₂ O Benzyl steht, die Benzyl-Gruppe abspaltet und die so erhaltene CH₂OH-Gruppe gewünschtenfalls verestert oder zum Aldehyd der Formel Ia oxidiert
c) eine Verbindung der Formel Ia 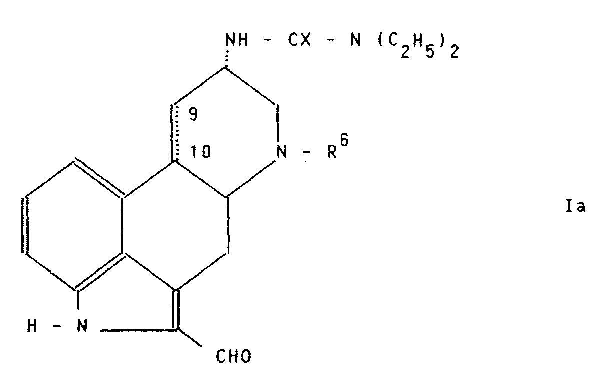 worin R⁶, X und 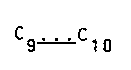 die obige Bedeutung besitzen,
   α) mit einem Wittig Reagenz umsetzt zu einer Verbindung der Formel I, worin R² einen gegebenenfalls mit Halogen substituierten C₂₋₁₀-Alkenylrest darstellt oder
   β) in eine Verbindung der Formel I mit R² in der Bedeutung CH(OH)R⁵ überführt, und gewünschtenfalls anschließend in eine Verbindung der Formel I, worin R² für C₂₋₁₀-Alkenyl oder C₂₋₁₀-Alkyl steht, überführt oder die Hydroxylgruppe verestert oder zum Keton oxidiert und gewünschtenfalls anschließend die so erhaltene Verbindung der Formel Ib 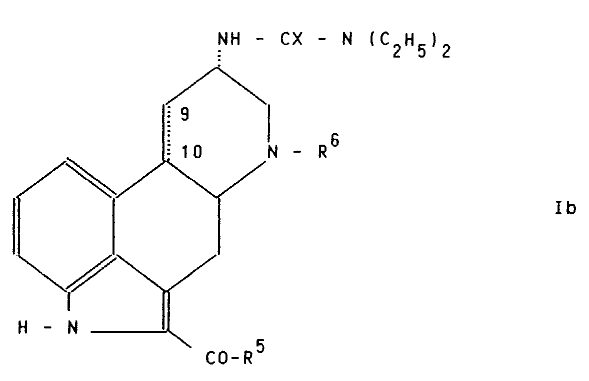
   worin R⁶, X, R⁵ und 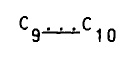 die obige Bedeutung haben, analog β) in CR¹² (OH)R⁵ überführt, worin R¹² die Bedeutung von C₁₋₉-Alkyl hat und gewünschtenfalls anschließend in eine Verbindung der Formel I, worin R² für C₂₋₁₀-Alkenyl oder C₂₋₁₀-Alkyl steht, überführt oder die Hydroxylgruppe verestert
   und gewünschtenfalls anschließend die nach α) oder β) erhaltenen ungesättigten Verbindungen reduziert zu Verbindungen der Formel I, worin R² für C₂₋₁₀-Alkyl steht,
d) eine Verbindung der Formel IV 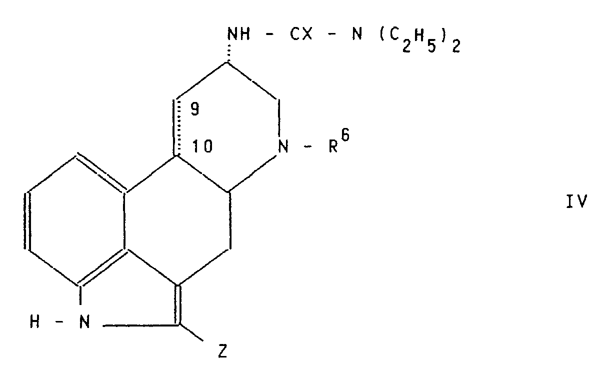 worin R⁶, X und 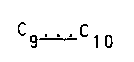 die obige Bedeutung besitzen und Z für Halogen oder Lithium steht, alkyliert oder alkenyliert zu einer Verbindung der Formel I, worin R² für C₁₋₁₀-Alkyl oder C₂₋₁₀-Alkenyl steht,
e) eine Verbindung der Formel V 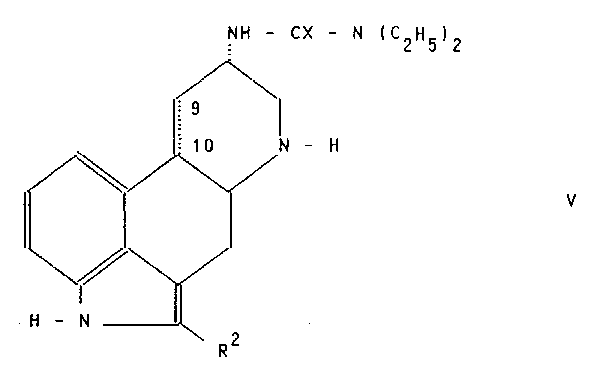 worin R², X und 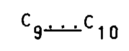 die obige Bedeutung haben, in 6-Stellung alkyliert und anschließend gewünschtenfalls die nach den Verfahren a-e) erhaltenen Verbindungen der Formel I mit X in der Bedeutung von Sauerstoff in die Thioharnstoffe überführt oder die 2,3-Doppelbindung reduziert oder die Säureadditionssalze bildet oder die Isomeren trennt.

Die Oxidation der Mannichbase der Formel II nach dem Verfahren a) kann beispielsweise bei Temperaturen von 0° C bis -40° C in inerten Lösungsmitteln wie Ethern mit tert.-Butylhypochlorit durchgeführt werden.

Als 5-6-gliedriger gesättigter Heterocyclus -NR⁷R⁸, der gegebenenfalls durch ein Sauerstoffatom unterbrochen sein kann, ist beispielsweise Piperidin, Morpholin oder Pyrrolidin geeignet.

Die Umsetzung der Verbindungen der Formel II und IIa nach dem Verfahren b) kann nach den in der EP-A-250 357 beschriebenen Methoden durchgeführt werden. Beispielsweise kann man in 2-Stellung des Ergolins das Quartärsalz einer disubst.-Aminomethyl-Verbindung mit Natriumborhydrid in polaren Lösungsmitteln wie Alkoholen zur 2-Methyl-Verbindung reduzieren oder mit nucleophilen Anionen substituieren zu Verbindungen der Formel I, worin R² CH₂YR³ oder CH₂CHR⁹COR¹⁰ bedeutet. Der nucleophile Austausch erfolgt nach Quartärnisierung der Aminomethylgruppe mit C₁₋₄-Alkylhalogeniden, beispielsweise mit Methyliodid, in einem inerten Lösungsmittel wie Alkoholen, Ethern oder chlorierten Kohlenwasserstoffen bei Raumtemperatur oder erhöhter Temperatur, wobei als nucleophile Anionen beispielsweise Mercaptide, Alkoholate und β-Dicarbonylverbindungen wie Malonester, Acetessigester und Acetylaceton umgesetzt werden können.

Die fakultativ nachfolgende Umwandlung der CH₂-O-Benzyl-Gruppe in die CH₂OH-Gruppe kann beispielsweise durch Abspaltung des Benzyl-Restes, mit Natrium oder Lithium in flüssigem Ammoniak und einem inerten Lösungsmittel wie Ether erfolgen.

Die Veresterung der Hydroxylgruppe kann jeweils nach den üblichen Methoden wie beispielsweise durch Umsetzung mit Säureanhydriden in Gegenwart der entsprechenden aliphatischen Carbonsäure und deren Alkalisalze in protischen Lösungsmitteln oder durch Umsetzung mit Säurechloriden in Gegenwart organischer Basen erfolgen.

Die Umwandlung der 2-Formyl-Verbindungen der Formel Ia in Verbindungen der Formel I, worin R₂ einen gegebenenfalls Halogen-substituierten C₂₋₁₀-Alkenylrest bedeutet, kann in einer Wittig-Reaktion erfolgen, indem man beispielsweise eine Verbindung der Formel Ia mit einem üblichen Wittig-Reagenz, wie beispielsweise Triphenylphosphin, Tetrahalogenmethan, Alkyl-triphenylphosphonium-halogenid oder Halogenalkyl-triphenylphosphintetrafluoroborat, gegebenenfalls nach Einführung üblicher Schutzgruppen an Protonendonatoren, umsetzt. Die Wittig-Reaktion kann in polaren Lösungsmitteln wie cyclischen und acyclischen Ethern, chlorierten Kohlenwasserstoffen, Dimethylformamid oder Dimethylsulfoxid bei Temperaturen von -50 °C bis zur Siedetemperatur des Reaktionsgemisches vorgenommen werden, wobei zur Erzeugung des Ylens starke Basen wie Alkalialkoholate, Lithiumorganyl u.a. zugesetzt werden.

Werden nach dem Verfahren c) Verbindungen der Formel I, worin R² CR¹²(OH)R⁵ bedeutet, hergestellt, so kann die Darstellung beispielsweise durch Grignardierung oder Lithium-alkylierung erfolgen. Die Grignardierung kann mit den üblichen Grignard-Reagenzen wie Alkylmagnesium-halogeniden in einem aprotischen Losungsmittel wie cyclischen und acyclischen Ethern bei tiefen Temperaturen (- 70 °C bis 0 °C) erfolgen. Die Umsetzung mit Alkyl-Lithium erfolgt unter analogen Bedingungen. Die Veresterung der Hydroxylgruppe kann nach den vorne beschriebenen Verfahren vorgenommen werden.

Die Oxidation zu einer 2-CHO nach Verfahren b) oder einer 2-CO-R⁵-Verbindung nach Verfahren c) kann jeweils analog dem in R.A. Jones et al. Synthetic Communications 16, 1799 (1986) beschriebenen Verfahren erfolgen, beispielsweise mit Braunstein in inerten Lösungsmitteln wie halogenierten Kohlenwasserstoffen bei Raumtemperatur.

Die fakultativ nachfolgende Einführung der Doppelbindung kann mit den üblichen Dehydratisierungsmethoden erfolgen, wie beispielsweise mit Sulfonaten oder Acetaten, wie Methansulfonsäurechlorid in polaren Lösungsmitteln, wie Ethern in Gegenwart einer Base und gegebenenfalls unter Erhitzen.

Enthält der Substituent R² eine exocyclische Doppelbindung, so kann diese in üblicher Weise reduziert werden zum entsprechenden Alkylderivat. Beispielsweise kann die Reduktion katalytisch mit Palladium/Kohle oder Raney-Nickel bei Raumtemperatur in einem aliphatischen Alkohol erfolgen oder die Reduktion kann mit Natrium in flüssigem Ammoniak in einem inerten Lösungsmittel wie cyclischen und acyclischen Ethern in Gegenwart eines Protonenspenders wie aliphatischen Alkoholen vorgenommen werden.

Enthalt der Substituent R² eine 2-Hydroxylgruppe, so kann diese beispielsweise durch Umsetzung mit NaBH₄ in Eisessig zum entsprechenden 2-Alkylderivat reduziert werden.

Das Verfahren d) kann beispielsweise nach den in der EP-A-160 842 beschriebenen Methoden wie Umsetzung von 2-Lithium-Ergolinen mit einem elektrophilen Reagenz oder Umsetzung von 2-Halogen-Ergolinen in Gegenwart eines Palladium-Katalysators und einer Base erfolgen.

Das Verfahren e) beschreibt die 6-Alkylierung von in 2-Stellung mit R² substituierten Ergolinen. Dieser Syntheseweg kann beispielsweise nach A. Cerny et al. Coll. Chech. Chem. Comm. 49 2828 (1984) erfolgen, indem man das 6-Cyan-Ergolin zur 6-H-Verbindung reduziert und diese anschließend mit entsprechenden Halogeniden alkyliert oder nach dem in der EP-21206 beschriebenen Verfahren.

Die Überführung der Harnstoffderivate in die Thioharnstoffderivate kann beispielsweise nach dem in der EP-A-217 730 beschriebenen Verfahren durch Umsetzung mit Phosphoroxychlorid und einem Thiolierungsmittel erfolgen. Die Reduktion der 2,3 Doppelbindung kann beispielsweise nach den in der EP-A-286575 beschriebenen Verfahren mit Organylsilanen in Gegenwart einer Säure, wie Trifluoressigsäure oder durch Natriumborhydrid erfolgen. Die Verbindungen der Formel I werden entweder als freie Basen oder in Form ihrer Säureadditionssalze isoliert.

Zur Bildung von Salzen wird eine Verbindung der Formel I beispielsweise in wenig Methanol oder Methylenchlorid gelöst und mit einer konzentrierten Lösung der gewünschten Säure versetzt.

Die Isomerengemische können nach üblichen Methoden wie beispielsweise Kristallisation, Chromatographie oder Salzbildung in die Diastereomeren bzw. E/Z-Isomeren aufgetrennt werden.

Die Erfindung betrifft auch die Verbindungen der Formel IIa, Ia und Ib, die wertvolle Zwischenprodukte zur Herstellung pharmakologisch wirksamer Verbindungen darstellen. Die Umwandlung der Zwischenprodukte in Wirksubstanzen erfolgt nach dem oben beschriebenen Verfahren.

Die Verbindungen der Formel V können durch eine Kupplungsreaktion von 2-Halogen-Ergolinen mit einer metallorganischen Verbindung unter Metallkatalyse erhalten werden (analog der Deutschen Patentanmeldung P 3824659). Bevorzugt sind Brom- und Jod-Derivate, die in einem inerten Lösungsmittel wie cyclischen und acyclischen Ethern, Kohlenwasserstoffen oder Dimethylformamid in Gegenwart eines Nickel- oder Palladiumkatalysators mit einer metallorganischen Verbindung bei Temperaturen bis zum Siedepunkt des Reaktionsgemisches umgesetzt werden. Als metallorganische Verbindungen sind C₁₋₁₀Alkyl-und C₂₋₁₀-Alkenyl-Me-Xₙ-Derivate geeignet, worin Me Zink, Magnesium, Zinn oder Bor, X Halogen, vorzugsweise Chlor oder Brom, C₁₋₄-Alkyl oder Hydroxy sind und n je nach Wertigkeit des Metallatoms ein -bis dreifach stehen kann.

Als Nickel-Katalysator wird beispielsweise das 1,3-Diphenylphosphinopropannickel-II-chlorid verwendet. Als Palladium-Katalysatoren können beispielsweise das Bis-tri-o-tolylphosphin-palladium-II-chlorid, das Bis-triphenylphosphin-palladium-II-chlorid, das Tetrakis-triphenylphosphin-palladium-II-chlorid und das 1,1'-Bis-diphenylphosphinoferrocen-palladium-II-chlorid verwendet werden.

Soweit die Herstellung der Ausgangsverbindungen nicht beschrieben wird, sind diese bekannt oder analog zu bekannten Verbindungen oder hier beschriebenen Verfahren herstellbar.

Die nachfolgenden Beispiele sollen das erfindungsgemäße Verfahren erläutern:

### Herstellung der Ausgangsverbindungen

1. Allgemeine Arbeitsvorschrift für die Mannich-Reaktion von 3-(6-Alkyl-8α-ergolinyl)-1,1-diethyl-harnstoffen.
   30 mmol Ergolin, 24.0g (0.19 mol) Morpholinhydrochlorid, 4.5g (0.15 mol) Paraformaldehyd und 220 ml trockenes Dimethylformamid werden in der angegebenen Reihenfolge zusammengegeben und die Mischung in einem auf 100°C vorgeheizten Ölbad 30 min gerührt. Zur Aufarbeitung gießt man die Reaktionslösung auf Eis, stellt mit 25%iger NH₃-Lösung alkalisch und extrahiert mit Toluol. Das nach Trocknen über Na₂SO₄ und Abziehen des Toluols i. Vak. erhaltene Rohprodukt wird in 100 ml Trifluoressigsäure gelöst und 30 min bei 60°C gerührt. Danach gießt man die Reaktionsmischung auf Eis und stellt mit 25%iger NH₃-Lösung alkalisch. Nach Extraktion mit Dichlormethan, Trocknen über Na₂SO₄ und Abziehen des Lösungsmittels i. Vak. erhält man ein dunkles Öl, das durch Chromatographie gereinigt wird.
   Folgende Verbindungen wurden dargestellt:
   **1,1-Diethyl-3-(2-morpholinomethyl-6-n-propyl-8α-ergolinyl)-harnstoff**
   Ausbeute nach Chromatographie (SiO₂, Dichlormethan/Methanol 97:3): 78 - 89%, [α]_{D} +11.3° (c=0.5 in Chloroform).
   **1,1 Diethyl-3-(2-morpholinomethyl-6-n-propyl-8α-ergolinyl)-harnstoff, L-Hydrogentartrat**
   Ausbeute: 84% (kristallin), [α]_{D} +1.6° (c=0.5 in Methanol).
   **1,1 Diethyl-3-(2-morpholinomethyl-6-cyclopropylmethyl-8α-ergolinyl)-harnstoff**
   Ausbeute nach Chromatographie (SiO₂, Dichlormethan/Methanol 99:1 - 97:3): 66%, [α]_{D} -0.8° (c=0.5 in Chloroform).
   **3-(6-Allyl-2-morpholinomethyl-8α-ergolinyl)-1,1-diethyl-harnstoff**
   Ausbeute nach Chromatographie (SiO₂, Dichlormethan/Methanol 97:3): 71 - 97% (36 - 59% kristallin), [α]_{D} +2.4° (c=0.5 in Chloroform).
   **1,1-Diethyl-3-(6-ethyl-2-morpholinomethyl-8α-ergolinyl)-harnstoff**
   Ausbeute nach Chromatographie (SiO₂, Dichlormethan/Methanol 99:1 - 95:5): 81% (46% kristallin), [α]_{D} +4.0° (c=0.5 in Chloroform).
   **3-(6-Cyano-9,10-didehydro-2-morpholinomethyl-8α-ergolinyl)-1,1-diethyl-harnstoff**
   Ausbeute nach Chromatographie (Essigester/Methanol 98:2 - 96:4): 46% (24% kristallin aus Essigester), [α]_{D} +301.6° (c=0.5 in Chloroform).
2. Allgemeine Arbeitsvorschrift für die Quarternisierung der Mannich-Verbindung.
   10 mmol Ergolin werden in ca. 200 ml Tetrahydrofuran gelöst und mit 4.0 ml (65 mmol) Methyliodid versetzt. Man läßt 17 - 22h bei Raumtemperatur rühren. Anschließend wird die Mischung, aus der bereits nach ca. 4h das Produkt auszufallen beginnt, im Eisbad gekühlt und tropfenweise mit 100 ml Diisopropylether versetzt. Nach 30 min bei 0°C wird das Quartärsalz abgesaugt. Das so erhaltene Material wird ohne Charakterisierung für die weiteren Umsetzungen verwendet.
   Folgende Verbindungen wurden hergestellt:
   **N-(8α-(3,3-diethylureido)-6-n-propyl-2-ergolinyl-methyl)-N-methyl-morpholiniumiodid**
   Ausbeute: 70 - 81%
   **N-(6-Allyl-8α-(3,3-diethylureido)-2-ergolinyl-methyl)-N-methyl-morpholiniumiodid**
   Ausbeute: 59 - 70%
   **N-(6-Cyclopropylmethyl-8α-(3,3-diethylureido)-2-ergolinylmethyl)-N-methyl-morpholiniumiodid**
   Ausbeute 64 - 80%
   **N-(6-Ethyl-8α-(3,3-diethylureido)-2-ergolinyl-methyl)-N-methyl-morpholiniumiodid**
   Ausbeute: 87%
   **N-(6-Cyano-9,10-didehydro-8α-(3,3-diethylureido)-2-ergolinyl-methyl)-N-methyl-morpholiniumiodid**
   Ausbeute: 82%
3. Bromierung von 1,1-Diethyl-3-(8α-ergolinyl)-harnstoff
   **3-(2-Brom-8α-ergolinyl)-1,1-diethyl-harnstoff**
   Herstellung und Daten siehe EP-56358
4. **1,1-Diethyl-3-(2-ethyl-8α-ergolinyl)-harnstoff**
   1.00 g (2.47 mmol) 3-(2-Brom-8α-ergolinyl)-1,1-diethyl-harnstoff wird in 10 ml Toluol gelöst, mit 90 mg (0.12 mmol) 1,1'-Bis-(diphenylphoshino)-ferrocen-palladium-II-chlorid versetzt und 15 min bei Raumtemperatur gerührt. Danach werden 5.5 ml Triethylboran (1 M Lösung in Tetrahydrofuran) und 2.5 ml 4 N Kalilauge hinzugefügt und die Mischung 4 h unter Rückfluß erhitzt. Die Reaktionsmischung wird mit 2 N Salzsäure angesäuert, mit konz. Ammoniak alkalisch gestellt und mit Dichlormethan extrahiert. Das nach Trocknung der organischen Phasen (Natriumsulfat) und Abziehen des Lösungsmittels i. Vak. erhaltene Rohprodukt wird an Kieselgel chromatographiert. (Dichlormethan/Methanol 98 : 2). Ausbeute nach Kristallisation (Essigester): 307 mg (35%)
   [α]_{D} +47.6° (c=0.5 in Chloroform),
5. Die Reduktion des Cyanamids erfolgte nach literaturbekannten Methoden ¹⁾.
   Folgende Verbindungen wurden aus den 6-Cyano-Derivaten hergestellt:
   **3-(9,10-Didehydro-2-methyl-8α-ergolinyl)-1,1-diethyl-harnstoff**
   **3-(9,10-Didehydro-2-methoxymethyl-8α-ergolinyl)-1,1-diethyl-harnstoff**
   **3-(9,10-Didehydro-2-methylthiomethyl-8α-ergolinyl)-1,1-diethyl-harnstoff**
   **3-(9,10-Didehydro-2-hydroxymethyl-8α-ergolinyl)-1,1-diethyl-harnstoff**
   **3-(9,10-Didehydro-2-ethyl-8α-ergolinyl)-1,1-diethyl-harnstoff**
6. **3-(6-Cyano-9,10-didehydro-2-methyl-8α-ergolinyl)-1,1-diethyl-harnstoff**
   Herstellung analog Beispiel 1
7. **3-(6-Cyano-9,10-didehydro-2-methylthiomethyl-8α-ergolinyl)-1,1-diethyl-harnstoff**
   Herstellung analog Beispiel 2
8. **3-(6-Cyano-9,10-didehydro-2-methoxymethyl-8α-ergolinyl)-1,1-diethyl-harnstoff**
   Herstellung analog Beispiel 4
9. **3-(6-Cyano-9,10-didehydro-2-hydroxymethyl-8α-ergolinyl)-1,1-diethyl-harnstoff**
   wurde durch Reduktion der 2-Formyl-verbindung erhalten.
10. **3-(6-Cyano-9,10-didehydro-2-hydroxyethyl-8α-ergolinyl)-1,1-diethyl-harnstoff**
   Herstellung analog Beispiel 8
11. **3-(6-Cyano-9,10-didehydro-2-ethyl-8α-ergolinyl)-1,1-diethyl-harnstoff**
   Herstellung analog Beispiel 13

¹⁾ A. Cerny et al. Collection Czechoslovak Chem. Commun. 1984, 49, 2828

### Beispiel 1

### Allgemeine Arbeitsvorschrift zur Reduktion der Quartärsalze mit Natriumborhydrid

1.00 mmol Ergolin werden in 25 ml trockenem Ethanol gelöst, mit 184.6 mg (5.00 mmol) pulverisiertem NaBH₄ versetzt und bei Raumtemperatur gerührt. Nach Ende der Umsetzung (DC) gießt man auf Eis und extrahiert mit Dichlormethan. Das nach Trocknen über Na₂SO₄ und Abziehen des Lösungsmittels i. Vak. erhaltene Rohprodukt wird durch Chromatographie oder Kristallisation gereinigt.

Folgende Verbindungen wurden hergestellt:
**3-(6-Cyclopropylmethyl-2-methyl-8α-ergolinyl)-1,1-diethyl-harnstoff**
Ausbeute nach Kristallisation (Essigester/Diisopropylether): 61%, [α]_{D} -5.2° (c=0.5 in Chloroform).
**3-(6-Allyl-2-methyl-8α-ergolinyl)-1,1-diethyl-harnstoff**
Ausbeute nach Chromatographie (SiO₂, Dichlormethan/Methanol 95:5): 67% (37% kristallin), [α]_{D} -2.0° (c=0.5 in Chloroform).
**1,1-Diethyl-3-(6-ethyl-2-methyl-8α-ergolinyl)-harnstoff**
Ausbeute nach Chromatographie (SiO₂, Dichlormethan/Methanol 97:3): 33% (13% kristallin), [α]_{D} +3.2° (c=0.5 in Chloroform).
**3-(6-n-Propyl-2-methyl-8α-ergolinyl)-1,1-diethyl-harnstoff**
Ausbeute nach Kristallisation (Essigester/Diisopropylether) : 65%, [α]_{D} + 34° (c = 0,5 in Pyridin)

### Beispiel 2

### Allgemeine Ärbeitsvorschrift zur Reaktion der Quartärsalze mit Natriummethanthiolat

1.00 mmol Ergolin werden in 50 ml Dichlormethan gelöst und mit 600 - 800 mg (8.56 - 11.14 mmol) NaSCH₃ versetzt. Man rührt 5 - 17h bei Raumtemperatur, falls nötig wird weiteres NaSCH₃ nachgegeben (DC). Zur Aufarbeitung gießt man auf Eis/NH₃-Lösung und extrahiert mit Dichlormethan. Das nach Trocknen über Na₂SO₄ und Abziehen des Lösungmittels i. Vak. erhaltene Material wird durch Chromatographie weiter gereinigt.

Folgende Verbindungen wurden hergestellt:
**1,1-Diethyl-3-(2-methylthiomethyl-6-n-propyl-8α-ergolinyl)-harnstoff**
Ausbeute nach Chromatographie (SiO₂, Essigester/Methanol 97:3): 43% (34% kristallin), [α]_{D} +12.8° (c=0.5 in Chloroform).
**3-(6-Cyclopropylmethyl-2-methylthiomethyl-8α-ergolinyl)-1,1-diethyl-harnstoff**
Ausbeute nach Chromatographie (SiO₂, Dichlormethan/Methanol 97:3): 46% (40% kristallin), [α]_{D} -2.4° (c=0.5 in Chloroform).
**3-(6-Allyl-2-methylthiomethyl-8α-ergolinyl)-1,1-diethyl-harnstoff**
Ausbeute nach Chromatographie (SiO₂, Essigester/Methanol 97:3): 51% (42% kristallin), [α]_{D} 0.0° (c=0.5 in Chloroform).
**1,1-Diethyl-3-(6-ethyl-2-methylthiomethyl-8α-ergolinyl)-harnstoff**
Ausbeute nach Chromatographie (SiO₂, Dichlormethan/ Methanol 97:3): 38% (24% kristallin), [α]_{D} +8.1° (c=0.5 in Chloroform).

### Beispiel 3

### Allgemeine Arbeitsvorschrift zur Reaktion der Quartärsalze mit Natriumbenzylat

1.17 g (0.05 mol) Natrium in kleinen Stücken werden unter Erwärmen in 40 ml trockenem Benzylalkohol aufgelöst. 10 mmol des Quartärsalzes werden in der minimalen Menge Benzylalkohol (25 - 50 ml) gelöst und langsam zu der auf Raumtemperatur abgekühlten Benzylat-Lösung getropft. Nach beendeter Zugabe läßt man noch bis zu 30 min rühren (DC). Zur Aufarbeitung wird die Reaktionslösung direkt chromatographiert (1.3 kg SiO₂, Dichlormethan). Der Benzylalkohol wird mit Dichlormethan eluiert (ca. 10 l). Durch Zusatz von 1 - 3% Methanol zum Laufmittel wird schließlich das Produkt erhalten.

Folgende Verbindungen wurden hergestellt:
**3-(2-Benzyloxymethyl-6-n-propyl-8α-ergolinyl)-1,1-diethyl-harnstoff**
Ausbeute: 93%.
**3-(2-Benzyloxymethyl-6-n-propyl-8α-ergolinyl)-1,1-diethyl-harnstoff, L-Hydrogentartrat**
Ausbeute: 43%, [α]_{D} +12.4° (c=0.5 in Methanol)
**3-(2-Benzyloxymethyl-6-cyclopropylmethyl-8α-ergolinyl)-1,1-diethyl-harnstoff**
Ausbeute: 43% (15% kristallin), [α]_{D} -8.2° (c=0.5 in Chloroform).
**3-(6-Allyl-2-benzyloxymethyl-8α-ergolinyl)-1,1-diethyl-harnstoff**
Ausbeute: 73% (31% kristallin), [α]_{D} -9.2° (c=0.5 in Chloroform).
**3-(2-Benzyloxymethyl-6-ethyl-8α-ergolinyl)-1,1-diethyl-harnstoff**
Ausbeute: 57%.
**3-(2-Benzyloxymethyl-6-ethyl-8α-ergolinyl)-1,1-diethyl-harnstoff, L-Hydrogentartrat**
Ausbeute: 67%, [α]_{D} +8.8° (c=0.5 in Methanol).

### Beispiel 4

### Allgemeine Arbeitsvorschrift zur Reaktion der Quartärsalze mit Natriummethanolat

Zu einer Lösung von 230 mg (10.0 mmol) Natrium in 20 ml trockenem Methanol wird eine Lösung von 2.0 mmol Quartärsalz in 20 ml Methanol bei 0°C tropfenweise zugegeben. Man rührt nach beendeter Zugabe 15 min bei 0°C und 30 min bei Raumtemperatur. Zur Aufarbeitung gießt man auf Eis/NH₃-Lösung und extrahiert mit Dichlormethan. Das nach Trocknung der organischen Phasen über Na₂SO₄ und Abziehen des Lösungsmittels i. Vak. erhaltene Rohprodukt wird durch Kristallisation oder Chromatographie gereinigt.

Folgende Verbindungen wurden hergestellt:
**1,1-Diethyl-3-(2-methoxymethyl-6-n-propyl-8α-ergolinyl)-harnstoff**
Ausbeute nach Chromatographie (SiO₂, Dichlormethan/Methanol 95:5): 44% (28% kristallin), [α]_{D} +5.8° (c=0.5 in Chloroform).
**3-(6-Cyclopropylmethyl-2-methoxymethyl-8α-ergolinyl)-1,1-diethyl-harnstoff**
Ausbeute nach Chromatographie (SiO₂, Dichlormethan/Methanol 97:3): 13% (8% kristallin), [α]_{D} -3.6° (c=0.5 in Chloroform).
**3-(6-Allyl-2-methoxymethyl-8α-ergolinyl)-1,1-diethyl-harnstoff**
Ausbeute nach Kristallisation (Essigester/Diisopropylether): 23%, [α]_{D} -3.0° (c=0.5 in Chloroform).
**1,1-Diethyl-3-(6-ethyl-2-methoxymethyl-8α-ergolinyl)-harnstoff**
Ausbeute nach Kristallisation (Essigester/Diisopropylether): 36%, [α]_{D} -0.9° (c=0.5 in Chloroform).

### Beispiel 5

### Allgemeine Arbeitsvorschrift zur Herstellung der Hydroxymethyl-Verbindungen aus den Benzylethern

2.00 mmol Ergolin werden in 25 ml trockenem Tetrahydrofuran gelöst und bei -70°C in 40 ml NH₃ getropft. Man gibt portionsweise 180 - 550 mg (8 - 24 mmol) Natrium in kleinen Stücken zu, wobei mit der Zugabe jeweils solange gewartet wird, bis das zuletzt zugegebene Natrium abreagiert ist. Nachdem das Edukt verbraucht ist (DC), wird Methanol zugegeben und das Kühlbad entfernt. Das nach dem Abdampfen des Ammoniaks verbliebene Lösungsmittel wird i. Vak. entfernt und der Rückstand in Wasser aufgenommen. Nach Extraktion mit Dichlormethan, Trocknen der organischen Phasen über Na₂SO₄ und Abziehen des Lösungsmittels i. Vak erhält man das Rohprodukt, welches durch Kristallisation oder Chromatographie weiter gereinigt wird.

Folgende Verbindungen wurden hergestellt:
**1,1-Diethyl-3-(2-hydroxymethyl-6-n-propyl-8α-ergolinyl)-harnstoff**
Ausbeute nach Chromatographie (Dichlormethan/Methanol 96:4): 88% (58% kristallin aus Essigester/Diisopropylether), [α]_{D} +14.2° (c=0.5 in Chloroform).
**3-(6-Allyl-2-hydroxymethyl-8α-ergolinyl)-1,1-diethyl-harnstoff**
Ausbeute nach Chromatographie (Dichlormethan/Methanol 97:3) 61% (36% kristallin aus Essigester/Diisopropylether), [α]_{D} +3.4° (c=0.5 in Chloroform).
Außerdem wurden 18% des 3-(6-Allyl-2-methyl-8α-ergolinyl)-1,1-diethyl-harnstoffs isoliert.
**1,1-Diethyl-3-(6-ethyl-2-hydroxymethyl-8α-ergolinyl)-harnstoff**
Ausbeute nach Chromatographie (Dichlormethan/Methanol 97:3 und 90:10) 69% (36% kristallin aus Essigester/Diisopropylether), [α]_{D} +9.8° (c=0.5 in Chloroform).
**3-(6-Cyclopropylmethyl-2-hydroxymethyl-8α-ergolinyl)-1,1-diethyl-harnstoff**
Ausbeute nach Chromatographie (Dichlormethan/Methanol 95:5) 44% (27% kristallin aus Essigester/Diisopropylether), [α]_{D} -0.4° (c=0.5 in Chloroform).

### Beispiel 6

### Allgemeine Arbeitsvorschrift für die Acetylierung der 2-Hydroxy-methylergoline

1.0 mmol Ergolin wird in 10 ml Eisessig gelöst. Man gibt 5 ml (0.05 mol) Acetanhydrid und 0.8 g (0.01 mol) wasserfreies Natriumacetat zu und läßt über Nacht bei Raumtemperatur rühren. Zur Aufarbeitung gibt man Eis zu, rührt 30 min und versetzt mit NH₃-Lösung. Nach Extraktion mit Dichlormethan, Trocknen der organischen Phasen über Na₂SO₄ und Abziehen des Lösungsmittels i. Vak erhält man das Rohprodukt, welches durch Kristallisation oder Chromatographie weiter gereinigt wird.

Folgende Verbindungen wurden hergestellt:
**3-(2-Acetoxymethyl-6-n-propyl-8α-ergolinyl)-1,1-diethyl-harnstoff**
Ausbeute nach Kristallisation: 34% (aus Essigester/Diisopropylether), [α]_{D} +3.2° (c=0.5 in Chloroform).
**3-(2-Acetoxymethyl-6-cyclopropylmethyl-8α-ergolinyl)-1,1-diethyl-harnstoff**
Ausbeute nach Kristallisation: 69% (aus Essigester/Diisopropylether), [α]_{D} -5.6° (c=0.5 in Chloroform).
**3-(2-Acetoxymethyl-6-ethyl-8α-ergolinyl)-1,1-diethyl-harnstoff**
Ausbeute nach Chromatographie (Dichlormethan/Methanol 95:5) 69% (48% kristallin aus Essigester), [α]_{D} -2.3° (c=0.5 in Chloroform).
**3-(2-Acetoxymethyl-9,10-didehydro-6-n-propyl-8α-ergolinyl)-1,1-diethyl-harnstoff**

### Beispiel 7

**3-(2-Acetoxymethyl-6-allyl-8α-ergolinyl)-1,1-diethyl-harnstoff**
213.6 mg (0.54 mmol) 3-(6-Allyl-2-hydroxymethyl-8α-ergolinyl)-1,1-diethyl-harnstoff wurden in 5 ml Pyridin gelöst und mit 10 ml (0.11 mol) Acetanhydrid versetzt. Nach 2h rühren bei Raumtemperatur wurde die Reaktionsmischung auf Eis gegeben, 1h gerührt und in der oben angegebenen Weise aufgearbeitet. Ausbeute nach Kristallisation: 64% (aus Essigester/Diisopropylether), [α]_{D} -7.2° (c=0.5 in Chloroform).

### Beispiel 8

### Allgemeine Arbeitsvorschrift für die Umsetzung der 2- Acyl-ergoline mit Methyl-Lithium

5.00 mmol Ergolin werden in 150 ml trockenem Tetrahydrofuran gelöst und auf -65°C gekühlt. Es werden 10 - 15 mmol Methyl-Lithium (1.6M Lösung in Ether) in 5 - 7 Portionen zugegeben. Nach der letzten Zugabe läßt man auftauen und rührt noch 30 min bei Raumtemperatur. Der Reaktionsverlauf wird dünnschichtchromatographisch verfolgt. Zur Aufarbeitung gießt man auf Eis, alkalisiert mit 25%iger NH₃-Lösung und extrahiert mit Essigester. Das nach Abziehen des Lösungsmittels erhaltene Rohprodukt wird chromatographiert.

Folgende Verbindungen wurden hergestellt:
**1,1-Diethyl-3-[2-(1-hydroxymethyl)-6-n-propyl-8α-ergolinyl]-harnstoff** (Diastereomerengemisch)
Ausbeute nach Chromatographie (Essigester/Methanol 97:3 bis 95:5): 66% (78% bez. auf Umsatz).
**3-(6-Allyl-2-(1-hydroxyethyl-8α-ergolinyl)-1,1-diethyl-harnstoff**
Ausbeute nach Chromatographie (Dichlormethan/Methanol 97:3): 28% (Diastereomerengemisch), (Das unpolarere Diastereomer wurde bei der Chromatographie rein erhalten und aus Essigester/Diisopropylether kristallisiert. Die angegebenen Daten beziehen sich auf dieses Isomere). [α]_{D} -42.8° (c=0.5 in Chloroform).
**1,1-Diethyl-3-[6-ethyl-2-(1-hydroxyethyl)-8α-ergolinyl)-harnstoff** (Diastereomerengemisch)
Ausbeute nach Chromatographie (Essigester/Methanol 95:5 bis 90:10): 74% (67% kristallin aus Essigester/Diisopropylether).
**(-)-3-[6-Cyclopropylmethyl-2-(1-hydroxyethyl)-8α-ergolinyl]-1,1-diethyl-harnstoff**
[α]_{D} -13.6° (c=0.5 in Chloroform), (unpolareres Isomer) und
**(+)-3-[6-Cyclopropylmethyl-2-(1-hydroxyethyl)-8α-ergolinyl]-1,1-diethyl-harnstoff**
[α]_{D} +16.7° (c=0.5 in Chloroform), (polareres Isomer)
Gesamtausbeute nach Chromatographie (Essigester/Methanol 97:3 und 95:5): 58% (66% bez. auf Umsatz)
**1,1-Diethyl-3-(2-(1-hydroxy-1-methyl-ethyl)-6-n-propyl-8α-ergolinyl)-harnstoff**
Ausbeute nach Chromatographie (Dichlormethan/Methanol 98:2): 73%, (47% kristallin aus Essigester), [α]_{D} +23.0° (c=0.5 in Chloroform).

### Beispiel 9

### Reaktionen mit Grignard-Reagenzien

a. 1.28 g (3.2 mmol) 1,1-Diethyl-3-(6-n-propyl-2-formyl-8α-ergolinyl)-harnstoff wurden mit 20 mmol Ethylmagnesiumbromid (10 ml 2M-Lösung in Ether) wie oben angegeben umgesetzt. Das Rohprodukt wurde zunächst an 2 hintereinandergeschalteten Fertigsäulen C (Fa. Merck) chromatographiert (Essigester/Methanol 97:3) Hierbei wurde das polarere Diastereomer in 36% Ausbeute rein isoliert.
   **1,1-Diethyl-3-[2-(1-hydroxy-n-propyl)-6-n-propyl-8α-ergolinyl]-harnstoff**
   [α]_{D} +26.1° (c=0.5 in Chloroform), (polareres Isomer) Die erhaltenen Mischfraktionen wurden nochmals chromatographiert (Dichlormethan/Methanol 98:2), wobei das unpolarere Diastereomer in 18% Ausbeute rein erhalten wurde.
   **1,1-Diethyl-3-[2-(1-hydroxy-n-propyl)-6-n-propyl-8α-ergolinyl]-harnstoff**
   [α]_{D} +6.2° (c=0.5 in Chloroform), (unpolareres Isomer) Außerdem erhielt man noch 21% Mischfraktion und 11% Edukt. Gesamtausbeute: 74% (82% bez. auf Umsatz).
b. 1.70 g (4.3 mmol) 1,1-Diethyl-3-(6-n-propyl-2-formyl-8α-ergolinyl)-harnstoff wurden mit 43 mmol n-Pentyl-magnesiumbromid (10 ml 2M-Lösung in Ether) wie oben angegeben umgesetzt.
   **1,1-Diethyl-3-[2-(1-hydroxy-n-hexyl)-6-n-propyl-8α-ergolinyl]-harnstoff** (Diastereomerengemisch)
   Ausbeute nach Chromatographie (Dichlormethan/Methanol 97:3): 25%

### Beispiel 10

### Allgemeine Arbeitsvorschrift zur Herstellung der 2-Alkenyl-Ergoline durch Eliminierung aus den 2-Hydroxyalkyl-Verbindungen

1.00 mmol Ergolin wird in 40 ml Tetrahydrofuran p.A. gelöst und mit 1.4 ml (10 mmol) Triethylamin versetzt. Nach Zugabe von 0.8 ml (10 mmol) Methansulfonsäurechlorid rührt man noch 30 min bei Raumtemperatur (DC nach 5 min). Anschließend gießt man die Mischung auf Eis, alkalisiert mit 25%iger NH₃-Lösung und extrahiert mit Essigester. Das nach Abdampfen des Lösungsmittels i. Vak. erhaltene Rohprodukt wird chromatographiert.

Folgende Verbindungen wurden hergestellt:
**1,1-Diethyl-3-(6-n-propyl-2-vinyl-8α-ergolinyl)-harnstoff**
Ausbeute nach Chromatographie (Dichlormethan/Methanol 97:3): 47% (11% kristallin aus Essigester/Diisopropylether), [α]_{D} +44.0° (c=0.5 in Chloroform).
**1,1-Diethyl-3-(6-ethyl-2-vinyl-8α-ergolinyl)-harnstoff**
Ausbeute nach Chromatographie (Essigester/Methanol 97:3): 41% (24% kristallin aus Essigester/Diisopropylether), [α]_{D} +34.2° (c=0.5 in Chloroform).
**3-(6-Allyl-2-vinyl-8α-ergolinyl)-1,1-diethyl-harnstoff**
Ausbeute nach Chromatographie (Aluminiumoxid/Essigester): 53% (31% kristallin aus Essigester/Diisopropylether), [α]_{D} +3.3° (c=0.5 in Chloroform).
**3-(6-Cyclopropylmethyl-2-vinyl-8α-ergolinyl)-1,1-diethyl-harnstoff**
Ausbeute nach Chromatographie (Aluminiumoxid/Essigester): 46% (24% kristallin aus Essigester/Diisopropylether), [α]_{D} +20.3° (c=0.5 in Chloroform).
**1,1-Diethyl-3-(2-isopropenyl-6-n-propyl-8 -ergolinyl)-harnstoff**
Ausbeute nach Kristallisation (Essigester/Diisopropylether): 46%, [α]_{D} +79.0° (c=0.5 in Chloroform).
1,1-Diethyl-3-(9,10-didehydro-6-n-propyl-2-vinyl-8α-ergolinyl)-harnstoff Ausbeute 59 %, [α]_{D} + 355 ° (c=0.5 in Chloroform)

### Beispiel 11

Herstellung der 2-Alkenyl-Ergoline durch Wittig-Reaktion Zu einer Suspension von 5.35 g (14 mmol) Fluormethyltriphenyl-phosphin-tetrafluoroborat ²⁾ in 120 ml trockenem Dioxan wurden 1.58 g (14 mmol) Kalium-*tert*-butanolat in drei Portionen in 2-3 minütigem Abstand zugesetzt. Man ließ 30 min bei Raumtemperatur rühren und tropfte dann eine Lösung von 560 mg (1.4 mmol) 1,1-Diethyl-3-(2-formyl-6-n-propyl-8α-ergolinyl)-harnstoff in 15 ml Dioxan zu. Nach 30 min (DC) wurde der Ansatz auf Eis gegossen und mit fester Citronensäure ein pH von 3 eingestellt. Man extrahierte mit Essigester und wusch die organische Phase mit 2N Citronensäure. Die vereinigten wässrigen Phasen wurden unter Kühlung mit 25%iger NH₃-Lösung alkalisiert und mit Dichlormethan extrahiert. Diese organischen Phasen wurden mit Wasser gewaschen, über MgSO₄ getrocknet und das Lösungsmittel i. Vak. entfernt. Nach Chromatographie (Fertigsäulen C; Merck, Diisopropylether/ Methanol 95:5) erhielt man eine Gesamtausbeute von 279 mg (48%) Fluorvinyl-Verbindung. Durch Kristallisation einzelner Fraktionen wurden beide Isomere rein erhalten.
**(E)-1,1-Diethyl-3-[2-(2-fluorethylenyl)-6-n-propyl-8α-ergolinyl]-harnstoff**
und
**(Z)-1,1-Diethyl-3-[2-(2-fluorethylenyl)-6-n-propyl-8α-ergolinyl]-harnstoff**
²⁾ Burton, D.J., Wiemers, D.M., J. Fluor. Chem. 27, 85 (1984).

### Beispiel 12

### Reduktion der 2-Alkenyl-ergoline

Ca. 15 ml Ammoniak werden bei -65°C mit 345 mg (15 mmol) Natrium versetzt und 5 min bei dieser Temperatur gerührt. Eine Lösung von 1.00 mmol Ergolin in 25 ml trockenem Tetrahydrofuran wird schnell zugetropft, wobei sich die tiefblaue Lösung nach halber Zugabe entfärbt. Nach 15 min gibt man 3 Mal je 0.2 ml Ethanol innerhalb von 5 min zu. Danach wird nach 10 min bei -65°C gerührt und dann durch Zugabe von 15 ml Ethanol und 15 ml Wasser die Reaktion beendet. Man läßt das Ammoniak abdampfen gibt auf Eis und extrahiert mit Dichlormethan. Das nach Abziehen das Lösungsmittels i. Vak. erhaltene Rohprodukt wird chromatographiert oder direkt kristallisiert.

Folgende Verbindungen wurden hergestellt:
**3-(6-Allyl-2-ethyl-8α-ergolinyl)-1,1-diethyl-harnstoff**
Ausbeute nach Chromatographie (Dichlormethan/Methanol 98:2 und 97:3): 76% (27% kristallin aus Essigester/Diisopropylether), [α]_{D} +2.9° (c=0.5 in Chloroform).
**1,1-Diethyl-3-(2,6-diethyl-8α-ergolinyl)-harnstoff**
Ausbeute nach Chromatographie (Essigester/Methanol 97:3): 85% (40% kristallin aus Essigester), [α]_{D} +6.2° (c=0.5 in Chloroform).

### Beispiel 13

### Reduktion der 2-Hydroxyalkyl-ergoline

2.00 mmol Ergolin werden in 25 ml Eisessig gelöst und unter Argon 1.0 g (26.4 mmol) Natriumborhydrid (halbe Tabletten) zugegeben. Nach Ende der Reaktion (DC) gießt man auf Eis, alkalisiert unter Kühlung mit 25%iger NH₃-Lösung und extrahiert mit Dichlormethan. Das nach Abziehen das Lösungsmittels i. Vak. erhaltene Rohprodukt wird chromatographiert oder direkt kristallisiert.

Folgende Verbindungen wurden hergestellt:
**3-(6-Cyclopropylmethyl-2-ethyl-8α-ergolinyl)-1,1-diethyl-harnstoff**
Ausbeute nach Chromatographie (Essigester/Methanol 97:3): 65% (56% kristallin aus Essigester/Diisopropylether), [α]_{D} -0.8° (c=0.5 in Chloroform).
**1,1-Diethyl-3-(2,6-di-n-propyl-8α-ergolinyl)-harnstoff**
Ausbeute nach Chromatographie (Essigester/Methanol 97:3): 63% (53% kristallin aus Essigester/Diisopropylether), [α]_{D} +14.4° (c=0.5 in Chloroform).
**1,1-Diethyl-3-(2-n-hexyl-6-n-propyl-8α-ergolinyl)-harnstoff**
Ausbeute nach Chromatographie (Essigester/Methanol 95:5): 70% (43% kristallin aus Essigester/Hexan), [α]_{D} +14.4° (c=0.5 in Chloroform).
**1,1-Diethyl-3-(2-isopropyl-6-n-propyl-8α-ergolinyl)-harnstoff**
Bei der Reduktion mit Natriumborhydrid entstand ein Gemisch der gewünschten Verbindung und 1,1-Diethyl-3-(2-isopropenyl-6-n-propyl-8α-ergolinyl)-harnstoff. Daher wurde die Rohmischung mit Na/NH₃ (s. Beispiel 12) nachreduziert. Ausbeute nach Kristallisation (Essigester): 28%, [α]_{D} +17.6° (c=0.5 in Chloroform).

### Beispiel 14

### N-Alkylierung von 2-Alkyl-ergolinen

Die Reaktion erfolgt nach literaturbekannten Methoden³⁾
Folgende Verbindungen wurden hergestellt:
**1,1-Diethyl-3-(2-ethyl-6-n-propyl-8α-ergolinyl)-harnstoff**
Ausbeute nach Chromatographie: 64% (34% kristallin aus *tert*.- Butylmethylether/Hexan), [α]_{D} +42.8° (c=0.5 in Pyridin).
**3-(9,10-Didehydro-2-methyl-6-n-propyl-8α-ergolinyl)-1,1-diethyl-harnstoff**, Ausbeute 63 %, [α]_{D} +284° c=0.5 in Chloroform).
**3-(6-Allyl-9,10-didehydro-2-methyl-8α-ergolinyl)-1,1-diethyl-harnstoff**, Ausbeute 53 %, [α]_{D} +308° (c=0.5 in Chloroform).
**3-(6-Cyclopropylmethyl-9,10-didehydro-2-methyl-8α-ergolinyl)-1,1-diethyl-harnstoff**, Ausbeute 42 %, [α]_{D} +293° (c=0.5 in Chloroform).
**3-(9,10-Didehydro-6-ethyl-2-methyl-8α-ergolinyl)-1,1-diethyl-harnstoff,** Ausbeute 69 %, [α]_{D} +311°(c=0.5 in Chloroform)
**3-(9,10-Didehydro-2-methoxymethyl-6-n-propyl-8α-ergolinyl)-1,1-diethyl-harnstoff**, Ausbeute 26 %, [α]_{D} +260° (c=0.5 in Chloroform).
**3-(9,10-Didehydro-2-methylthiomethyl-6-n-propyl-8α-ergolinyl)-1,1-diethyl-harnstoff**, Ausbeute 50 % als Hydrogentartrat, [α]_{D} +169° (c=0,5 in Pyridin)
**3-(9,10-Didehydro-2-hydroxymethyl-6-n-propyl-8α-ergolinyl)-1,1-diethyl-harnstoff**
**3-(9,10-Didehydro-2-ethyl-6-n-propyl-8α-ergolinyl)-1,1-diethyl-harnstoff**, Ausbeute 38 %, [α]_{D} + 287° (c=0.5 in Chloroform).
³⁾ EP-21206
⁴⁾R.A. Jones et al. Synthetic Communications 16, 1799 (1986)

### Beispiel 15

### Allgemeine Arbeitsvorschrift zur Oxidation der 2-Hydroxyalkyl-ergoline mit Braunstein⁴⁾

1.0 mmol Ergolin wird in 20 ml Dichlormethan p.A. gelöst und 865 mg (10 mmol) MnO₂ (gefällt aktiv der Fa. Merck) zugegeben. Man läßt über Nacht bei Raumtemperatur rühren, filtriert über Aluminiumoxid/Celite und wäscht gut mit Dichlormethan und Essigester nach. Das reine Produkt wird durch Kristallisation oder Chromatographie erhalten.

Folgende Verbindungen wurden hergestellt:
**3-(6-Allyl-2-formyl-8α-ergolinyl)-1,1-diethyl-harnstoff**
Ausbeute nach Chromatographie (Dichlormethan/Methanol 97:3): 94% (65% kristallin aus Essigester/Diisopropylether), [α]_{D} +21.3° (c=0.5 in Chloroform)
**3-(6-Cyclopropylmethyl-2-formyl-8α-ergolinyl)-1,1-diethyl-harnstoff**
Ausbeute nach Kristallisation: 70% (aus Essigester/Diisopropylether), [α]_{D} +27.0° (c=0.5 in Chloroform).
**3-(2-Acetyl-6-n-propyl-8α-ergolinyl)-1,1-diethyl-harnstoff**
Ausbeute: 88% (57% kristallin aus Essigester), [α]_{D} +20.3° (c=0.5 in Chloroform).

### Beispiel 16

### Allgemeine Arbeitsvorschrift für die Oxidation der Mannich-Verbindungen mit tert-Butylhypochlorid

10.0 mmol Ergolin werden in 160 ml Tetrahydrofuran p.A. gelöst, auf -40°C gekühlt und 1.6 - 3.2 ml (11.5 - 23.0 mmol) Triethylamin zugesetzt. Anschließend wird eine Lösung von 1.5 ml (12.3 mmol) *tert*-Butylhypochlorid in 25 ml Tetrahydrofuran zügig zugetropft. Nach 5 min wird die Mischung dünnschichtchromatographisch kontrolliert. Falls sich noch Edukt nachweisen läßt, wird noch Hypochlorid nachgegeben. 30 min nach der letzten Zugabe (DC) gießt man den Ansatz auf Eis, alkalisiert mit 25%iger NH₃-Lösung und extrahiert mit Essigestar. Das nach Abziehen des Lösungsmittels i. Vak. erhaltene Rohprodukt muß chromatographiert werden.

Folgende Verbindungen wurden hergestellt:
**1,1-Diethyl-3-(2-formyl-6-n-propyl-8α-ergolinyl)-harnstoff**
Ausbeute nach Chromatographie (Essigester/Methanol 98:2 bis 95:5): 36 - 64%
**3-(6-Allyl-2-formyl-8α-ergolinyl)-1,1-diethyl-harnstoff**
Ausbeute nach Chromatographie (Essigester/Methanol 97:3): 32 - 65%
**3-(6-Cyclopropylmethyl-2-formyl-8α-ergolinyl)-1,1-diethyl-harnstoff**
Ausbeute nach Filtration über Aluminiumoxid Akt. III (Essigester): 82%
**1,1-Diethyl-3-(6-ethyl-2-formyl-8α-ergolinyl)-harnstoff**
Ausbeute nach Chromatographie (Dichlormethan/Methanol 97:3): 28 - 62%, (kristallisiert aus Essigester/Diisopropylether), [α]_{D} +43.8° (c=0.5 in Chloroform).
**3-(6-Cyano-9,10-didehydro-2-formyl-8α-ergolinyl)-1,1-diethyl-harnstoff**, Ausbeute 63 %.

### Beispiel 17

### Allgemeine Arbeitsvorschrift zur Darstellung der Thioharnstoffe aus den Harnstoffen.

Bei -20° löst man in 5 ml Dichlormethan nacheinander 0,13 ml frisch destilliertes Phosphoroxychlorid (1,4 mmol) und 0,23 mmol Ergolinylharnstoff. Die Mischung wird über Nacht bei Raumtemperatur gerührt, dann die flüchtigen Anteile sorgfältig im Vakuum abgezogen und der Rückstand in 10 ml Acetonitril gelöst. Dazu gibt man bei Raumtemperatur die Lösung von 205 mg Kaliumethylxanthogenat (1,4 mmol) in 20 ml Acetonitril, rührt 2 Stunden bei Raumtemperatur und versetzt mit Eis und konz. Ammoniaklösung. Die Mischung wird mit Dichlormethan extrahiert, die organischen Phasen mit Natriumsulfat mit Essigester chromatographiert und aus Essigester/Diisopropylether kristallisiert.

Folgende Verbindungen wurden hergestellt:
3-(9,10-Didehydro-2-ethyl-6-n-propyl-8α-ergolinyl)-diethyl-thioharnstoff Ausbeute 15 %, [α]_{D} = +373° (0,25 % in Chloroform)
3-(9,10-Didehydro-2-methyl-6-n-propyl-8α-ergolinyl)-diethyl-thioharnstoff Ausbeute 39 %, [α]_{D} = + 419 ° (0,5 % in Chloroform)
1,1-Diethyl-3-(2,6-di-n-propyl-8α-ergolinyl)-thioharnstoff Ausbeute 51 %, [α]_{D} = + 91 ° (0,25 % in Chloroform)
1,1-Diethyl-3-(2-methyl-6-n-propyl-8α-ergolinyl)-thioharnstoff Ausbeute 61 %, [α]_{D} = + 49° (0,5 % in Chloroform).

### Beispiel 18

### 1,1-Diethyl-3-(9,10-didehydro-2,3β-dihydro-2β-methyl-6-n-propyl-8α-ergolinyl)-harnstoff

Man löst 880 mg 3-(9,10-Didehydro-2-methyl-6-n-propyl-8α-ergolinyl)-1,1-diethylharnstoff (2,3 mmol) in 30 ml Trifluoressigsäure und gibt bei Raumtemperatur 1,48 ml Triethylsilan in drei gleichen Portionen im Abstand von 5 Minuten zu. Dann rührt man 60 Minuten, fügt erst Eis und anschließend 25%ige Ammoniaklösung unter Kühlen zu und schüttelt die alkalische Lösung mit Dichlormethan aus. Die organischen Phasen werden getrocknet und eingedampft, Ausbeute nach Chromatographie 378 mg. Durch Kristallisation aus Essigester/Diisopropylether können 106 mg erhalten werden (12 % d. Th.), [α]_{D} = + 204° (0,5 % in Chloroform).
1,1-Diethyl-3-(2,3β-dihydro-2-methyl-6-n-propyl-8α-ergolinyl)-harnstoff Ausbeute 63 %
1,1-Diethyl-3-(2,3β-dihydro-6-ethyl-2-methyl-8α-ergolinyl)-harnstoff Ausbeute 34 %, [α]_{D} = + 50° (0.5 % in Chloroform)
1,1-Diethyl-3-(2,3β-dihydro-2,6-di-n-propyl-8α-ergolinyl)-harnstoff Ausbeute 33 %, [α]_{D} = + 63° (0,5% in Chloroform)
1,1-Diethyl-3-(2,3β-dihydro-6-ethyl-2-methyl-8α-ergolinyl)-thioharnstoff Ausbeute 28 %, [α]_{D} = + 70° (0,5% in Chloroform)

## Patentansprüche

1. Verbindungen der Formel I 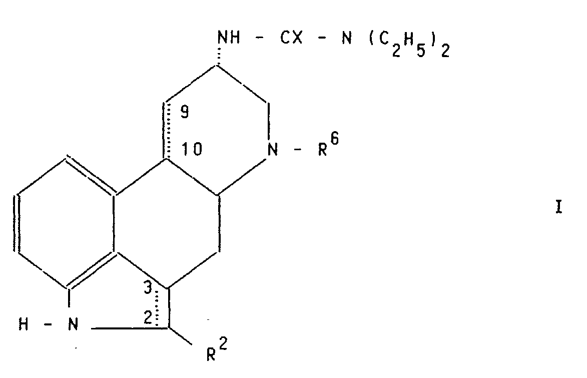 worin 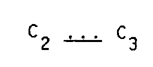 und 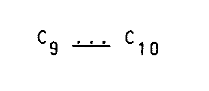 eine Einfach- oder Doppelbindung,
X Sauerstoff oder Schwefel,
R² C₁₋₁₀-Alkyl, gegebenenfalls mit Halogen substituiertes C₂₋₁₀-Alkenyl, CH₂YR³, CR¹²(OR⁴)R⁵, CH₂-CHR⁹-COR¹⁰ oder COR¹²
worin Y Sauerstoff oder Schwefel, R³ Wasserstoff, C₁₋₄-Alkyl, Phenyl, C₂₋₅-Acyl oder Phenyl-C₁₋₄-Alkyl, R⁴ Wasserstoff oder C₂₋₅-Acyl, R⁵ C₁₋₉-Alkyl, R⁹ COCH₃ oder COO-C₁₋₄-Alkyl, R¹⁰ C₁₋₄-Alkyl oder O-C₁₋₄-Alkyl, R¹² Wasserstoff oder C₁₋₉ Alkyl bedeuten und
R⁶ C₂₋₁₀-Alkyl, C₃₋₁₀-Alkenyl oder C₃₋₇-Cycloalkyl-C₁₋₃-alkyl sind,
sowie deren Säureadditionssalze, Isomeren und Isomerengemische.

2. 3-(6-Cyclopropylmethyl-2-methyl-8α-ergolinyl)-1,1-diethyl-harnstoff
3-(6-Allyl-2-methyl-8α-ergolinyl)-1,1-diethyl-harnstoff
1,1-Diethyl-3-(6-ethyl-2-methyl-8α-ergolinyl)-harnstoff
1,1-Diethyl-3-(2-methyl-6-n-propyl-8α-ergolinyl)-harnstoff
1,1-Diethyl-3-(2-methylthiomethyl-6-n-propyl-8α-ergolinyl)-harnstoff
3-(9,10-Didehydro-2-methylthiomethyl-6-n-propyl-8α-ergolinyl)-1,1-diethyl-harnstoff
3-(6-Allyl-2-methylthiomethyl-8α-ergolinyl)-1,1-diethyl-harnstoff
1,1-Diethyl-3-(6-ethyl-2-methylthiomethyl-8α-ergolinyl)-harnstoff
3-(2-Benzyloxymethyl-6-n-propyl-8α-ergolinyl)-1,1-diethyl-harnstoff
1,1-Diethyl-3-(2-methoxymethyl-6-n-propyl-8α-ergolinyl)-harnstoff
3-(9,10-Didehydro-2-methoxymethyl-6-n-propyl-8α-ergolinyl)-1,1-diethyl-harnstoff
3-(6-Allyl-2-methoxymethyl-8α-ergolinyl)-1,1-diethyl-harnstoff
1,1-Diethyl-3-(6-ethyl-2-methoxymethyl-8α-ergolinyl)-harnstoff
1,1-Diethyl-3-(2-hydroxymethyl-6-n-propyl-8α-ergolinyl)-harnstoff
3-(9,10-Didehydro-2-methyl-6-n-propyl-8α-ergolinyl)-1,1-diethyl-harnstoff
3-(9,10-Didehydro-2-ethyl-6-n-propyl-8α-ergolinyl)-1,1-diethyl-harnstoff
3-(9,10-Didehydro-6-ethyl-2-methyl-8α-ergolinyl)-1,1-diethyl-harnstoff
1,1-Diethyl-3-(2-methyl-6-n-propyl-8α-ergolinyl)-thioharnstoff
1,1-Diethyl-3-(2,3β-dihydro-6-ethyl-2-methyl-8α-ergolinyl)-thioharnstoff
1,1-Diethyl-3-(2-acetoxymethyl-6-n-propyl-8α-ergolinyl)-harnstoff
1,1-Diethyl-3-[2-(1-hydroxyethyl)-6-n-propyl-8α-ergolinyl]-harnstoff
1,1-Diethyl-3-(6-ethyl-2-vinyl-8α-ergolinyl)-harnstoff
1,1-Diethyl-3-(9,10-didehydro-6-n-propyl-2-vinyl-8α-ergolinyl)-harnstoff
1,1-Diethyl-3-(6-cyclopropylmethyl-2-vinyl-8α-ergolinyl)-harnstoff
1,1-Diethyl-3-(6-n-propyl-2-vinyl-8α-ergolinyl)-1,1-diethyl-harnstoff
1,1-Diethyl-3-(2-ethyl-6-n-propyl-8α-ergolinyl)-harnstoff
1,1-Diethyl-3-(9,10-didehydro-2-methyl-6-n-propyl-8α-ergolinyl)-thioharnstoff
1,1-Diethyl-3-(9,10-didehydro-2,3β-dihydro-2β-methyl-6-n-propyl-8α-ergolinyl)-harnstoff
1,1-Diethyl-3-(2,3β-dihydro-2-methyl-6-n-propyl-8α-ergolinyl)-harnstoff
1,1-Diethyl-3-(2,3β-dihydro-2,6-di-n-propyl-8α-ergolinyl)-harnstoff
(E)-1,1-Diethyl-3-[2-(2-fluorethylenyl)-6-n-propyl-8α-ergolinyl]-harnstoff
(Z)-1,1-Diethyl-3-[2-(2-fluorethylenyl)-6-n-propyl-8α-ergolinyl]-harnstoff
3-(6-Allyl-2-ethyl-8α-ergolinyl)-1,1-diethyl-harnstoff
1,1-Diethyl-3-(2,6-diethyl-8α-ergolinyl)-harnstoff
3-(6-Cyclopropylmethyl-2-ethyl-8α-ergolinyl)-1,1-diethyl-harnstoff
1,1-Diethyl-3-(2,6-di-n-propyl-8α-ergolinyl)-harnstoff
1,1-Diethyl-3-(2-n-hexyl-6-n-propyl-8α-ergolinyl)-harnstoff

3. Arzneimittel auf Basis der Verbindungen gemäß den Ansprüchen 1 und 2.

4. Verfahren zur Herstellung von Verbindungen der Formel I gemäß Anspruch 1, indem man
a) eine Verbindung der Formel II 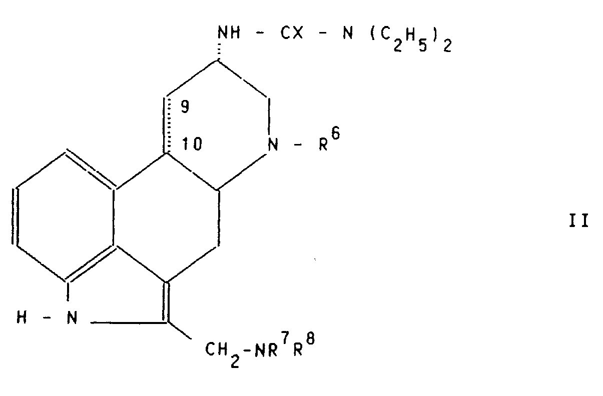 worin R⁶, X und 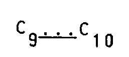 die obige Bedeutung haben und R⁷ und R⁸ jeweils C₁₋₆-Alkyl oder gemeinsam mit dem Stickstoffatom einen 5-6 gliedrigen gesättigten Heterocyclus, der gegebenenfalls durch ein Sauerstoffatom unterbrochen sein kann, bedeuten, zum Aldehyd der Formel Ia oxidiert 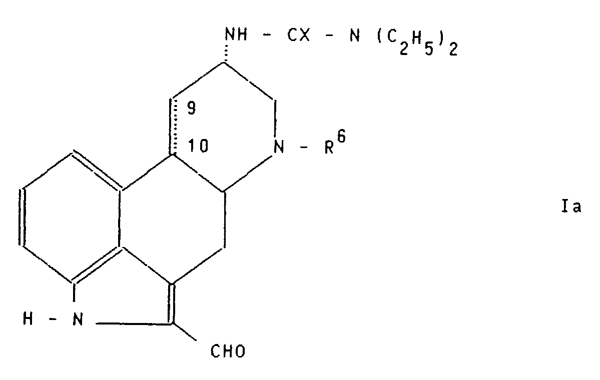 worin R⁶, X und 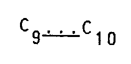 die obige Bedeutung besitzen,
b) das quartäre Salz der Formel IIa 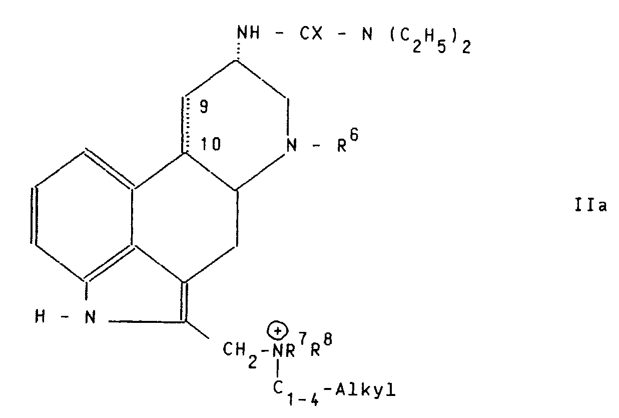 worin R⁶, R⁷, R⁸, X und 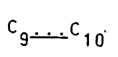 die obige Bedeutung haben, reduziert zu einer Verbindung der Formel I, worin R² Methyl ist oder mit einem nucleophilen Anion umsetzt und gewünschtenfalls in einer so erhaltenen Verbindung der Formel I, worin R² für CH₂ O Benzyl steht, die Benzyl-Gruppe abspaltet und die so erhaltene CH₂OH-Gruppe gewünschtenfalls verestert oder zum Aldehyd der Formel IA oxidiert
c) eine Verbindung der Formel Ia
α) mit einem Wittig Reagenz umsetzt zu einer Verbindung der Formel I, worin R² einen gegebenenfalls mit Halogen substituierten C₂₋₁₀-Alkenylrest darstellt oder
β) in CH(OH)R⁵ überführt, und gewünschtenfalls anschließend in eine Verbindung der Formel I, worin R² für C₂₋₁₀-Alkenyl oder C₂₋₁₀-Alkyl steht, überführt oder die Hydroxylgruppe verestert oder zum Keton oxidiert und gewünschtenfalls anschließend die so erhaltene Verbindung der Formel Ib 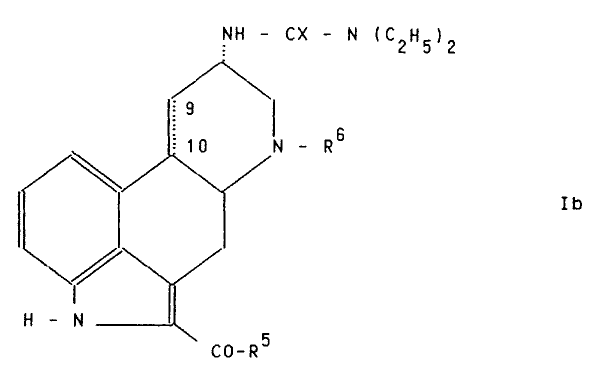
worin R⁶, X, R⁵ und 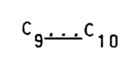 die obige Bedeutung haben, analog β) in CR¹² (OH)R⁵ überführt, worin R¹² die Bedeutung C₁₋₉ Alkyl hat und gewünschtenfalls anschließend in eine Verbindung der Formel I, worin R² für C₂₋₁₀-Alkenyl oder C₂₋₁₀-Alkyl steht, überführt oder die Hydroxylgruppe verestert
und gewünschtenfalls anschließend die nach α) oder β) erhaltenen ungesättigten Verbindungen reduziert zu Verbindungen der Formel I, worin R² für C₂₋₁₀-Alkyl steht,
d) eine Verbindung der Formel IV 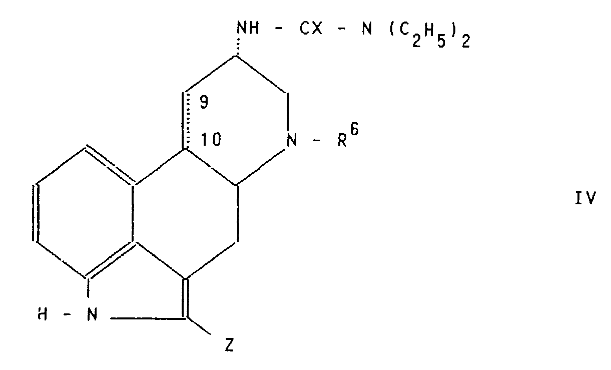 worin R⁶, X und C₉-C₁₀ die obige Bedeutung besitzen, und Z für Halogen oder Lithium steht, alkyliert oder alkenyliert zu einer Verbindung der Formel I, worin R₂ für C₁₋₁₀-Alkyl oder C₂₋₁₀-Alkenyl steht,
e) eine Verbindung der Formel V 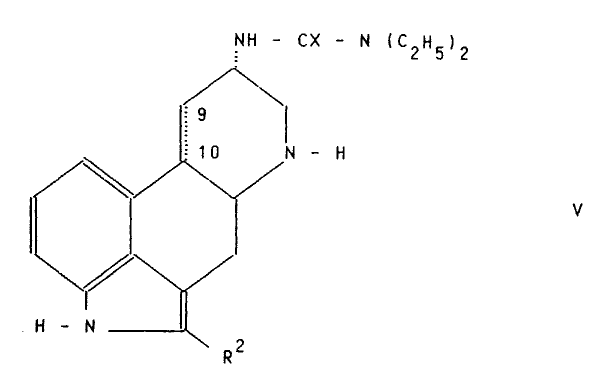 worin R², X und 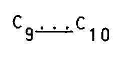 die obige Bedeutung besitzen, in 6-Stellung alkyliert und anschließend gewünschtenfalls die nach Verfahren a-e) erhaltenen Verbindungen der Formel I mit X in der Bedeutung von Sauerstoff in die Thioharnstoffe überführt oder die 2,3-Doppelbindung reduziert oder die Saureadditionssalze bildet oder die Isomeren trennt.

5. Zwischenverbindungen der Formel IIa, Ia, Ib und gemäß Anspruch 4.

## Claims

1. Compounds of formula I 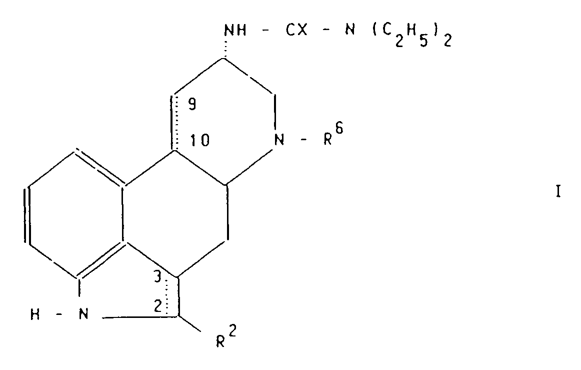 wherein 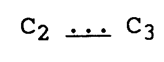 and 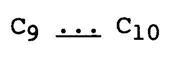 are a single or a double bond,
X is oxygen or sulfur,
R² is C₁₋₁₀alkyl, optionally halogen-substituted C₂₋₁₀alkenyl, CH₂YR³, CR¹²(OR⁴)R⁵, CH₂-CHR⁹-COR¹⁰ or COR¹²
wherein Y is oxygen or sulfur, R³ is hydrogen, C₁₋₄alkyl, phenyl, C₂₋₅acyl or phenyl-C₁₋₄alkyl, R⁴ is hydrogen or C₂₋₅acyl, R⁵ is C₁₋₉alkyl, R⁹ is COCH₃ or COO-C₁₋₄alkyl, R¹⁰ is C₁₋₄alkyl or O-C₁₋₄alkyl, R¹² is hydrogen or C₁₋₉alkyl and
R⁶ is C₂₋₁₀alkyl, C₃₋₁₀alkenyl or C₃₋₇cycloalkyl-C₁₋₃alkyl,
and the acid addition salts, isomers and isomeric mixtures thereof.

2. 3-(6-Cyclopropylmethyl-2-methyl-8α-ergolinyl)-1,1-diethylurea
3-(6-allyl-2-methyl-8α-ergolinyl)-1,1-diethylurea
1,1-diethyl-3-(6-ethyl-2-methyl-8α-ergolinyl)-urea
1,1-diethyl-3-(2-methyl-6-n-propyl-8α-ergolinyl)-urea
1,1-diethyl-3-(2-methylthiomethyl-6-n-propyl-8α-ergolinyl)-urea
3-(9,10-didehydro-2-methylthiomethyl-6-n-propyl-8α-ergolinyl)-1,1-diethylurea
3-(6-allyl-2-methylthiomethyl-8α-ergolinyl)-1,1-diethylurea
1,1-diethyl-3-(6-ethyl-2-methylthiomethyl-8α-ergolinyl)-urea
3-(2-benzyloxymethyl-6-n-propyl-8α-ergolinyl)-1,1-diethylurea
1,1-diethyl-3-(2-methoxymethyl-6-n-propyl-8α-ergolinyl)-urea
3-(9,10-didehydro-2-methoxymethyl-6-n-propyl-8α-ergolinyl)-1,1-diethylurea
3-(6-allyl-2-methoxymethyl-8α-ergolinyl)-1,1-diethylurea
1,1-diethyl-3-(6-ethyl-2-methoxymethyl-8α-ergolinyl)-urea
1,1-diethyl-3-(2-hydroxymethyl-6-n-propyl-8α-ergolinyl)-urea
3-(9,10-didehydro-2-methyl-6-n-propyl-8α-ergolinyl)-1,1-diethylurea
3-(9,10-didehydro-2-ethyl-6-n-propyl-8α-ergolinyl)-1,1-diethylurea
3-(9,10-didehydro-6-ethyl-2-methyl-8α-ergolinyl)-1,1-diethylurea
1,1-diethyl-3-(2-methyl-6-n-propyl-8α-ergolinyl)-thiourea
1,1-diethyl-3-(2,3β-dihydro-6-ethyl-2-methyl-8α-ergolinyl)-thiourea
1,1-diethyl-3-(2-acetoxymethyl-6-n-propyl-8α-ergolinyl)-urea
1,1-diethyl-3-[2-(1-hydroxyethyl)-6-n-propyl-8α-ergolinyl]-urea
1,1-diethyl-3-(6-ethyl-2-vinyl-8α-ergolinyl)-urea
1,1-diethyl-3-(9,10-didehydro-6-n-propyl-2-vinyl-8α-ergolinyl)-urea
1,1-diethyl-3-(6-cyclopropylmethyl-2-vinyl-8α-ergolinyl)-urea
1,1-diethyl-3-(6-n-propyl-2-vinyl-8α-ergolinyl)-1,1-diethylurea
1,1-diethyl-3-(2-ethyl-6-n-propyl-8α-ergolinyl)-urea
1,1-diethyl-3-(9,10-didehydro-2-methyl-6-n-propyl-8α-ergolinyl)-thiourea
1,1-diethyl-3-(9,10-didehydro-2,3β-dihydro-2β-methyl-6-n-propyl-8α-ergolinyl)-urea
1,1-diethyl-3-(2,3β-dihydro-2-methyl-6-n-propyl-8α-ergolinyl)-urea
1,1-diethyl-3-(2,3β-dihydro-2,6-di-n-propyl-8α-ergolinyl)-urea
(E)-1,1-diethyl-3-[2-(2-fluoroethylenyl)-6-n-propyl-8α-ergolinyl]-urea
(Z)-1,1-diethyl-3-[2-(2-fluoroethylenyl)-6-n-propyl-8α-ergolinyl]-urea
3-(6-allyl-2-ethyl-8α-ergolinyl)-1,1-diethylurea
1,1-diethyl-3-(2,6-diethyl-8α-ergolinyl)-urea
3-(6-cyclopropylmethyl-2-ethyl-8α-ergolinyl)-1,1-diethylurea
1,1-diethyl-3-(2,6-di-n-propyl-8α-ergolinyl)-urea
1,1-diethyl-3-(2-n-hexyl-6-n-propyl-8α-ergolinyl)-urea.

3. Medicaments based on the compounds according to claims 1 and 2.

4. Process for the preparation of compounds of formula I according to claim 1, in which
a) a compound of formula II 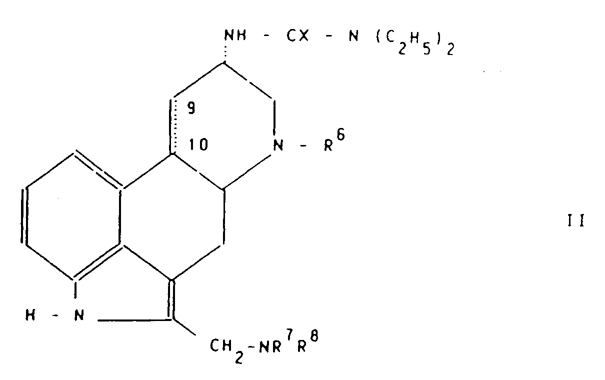 wherein R⁶, X and 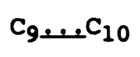 are as defined above and R⁷ and R⁸ are each C₁₋₆alkyl or together with the nitrogen atom are a 5- or 6-membered saturated heterocycle that may optionally be interrupted by an oxygen atom, is oxidised to the aldehyde of formula Ia 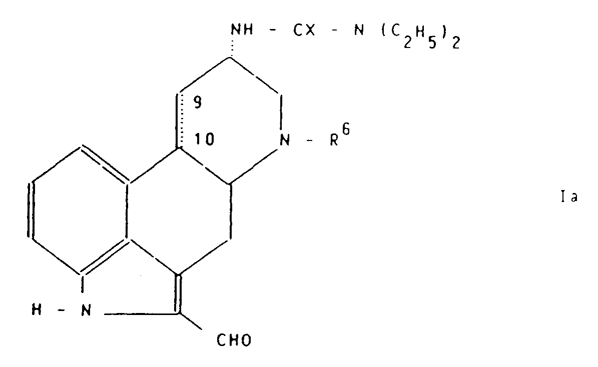 wherein R⁶, X and 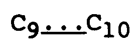 are as defined above,
b) the quaternary salt of formula IIa 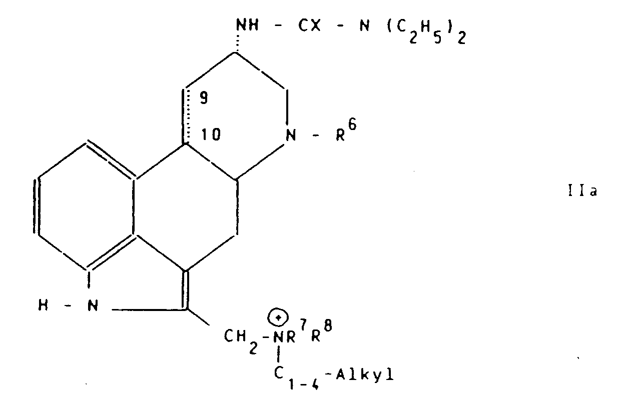 wherein R⁶, R⁷, R⁸, X and 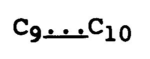 are as defined above, is reduced to a compound of formula I wherein R² is methyl or is reacted with a nucleophilic anion and, if desired, in a compound of formula I thus obtained in which R² is CH₂Obenzyl, the benzyl group is removed and the resulting CH₂OH group is, if desired, esterified or oxidised to the aldehyde of formula IA,
c) a compound of formula Ia is
α) reacted with a Wittig reagent to form a compound of formula I wherein R² is an optionally halogen-substituted C₂₋₁₀alkenyl radical or
β) converted into CH(OH)R⁵ and, if desired, is then converted into a compound of formula I wherein R² is C₂₋₁₀alkenyl or C₂₋₁₀alkyl, or the hydroxy group is esterified or oxidised to the ketone and then, if desired, the resulting compound of formula Ib 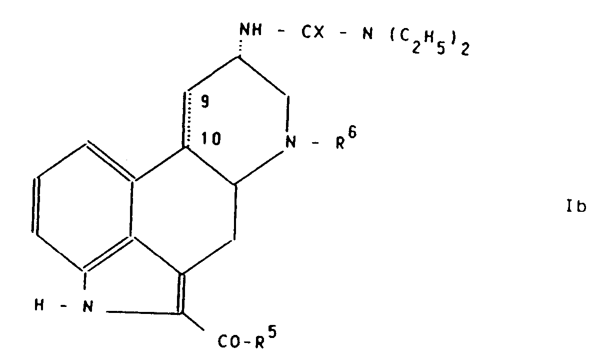
wherein R⁶, X, R⁵ and 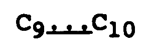 are as defined above, is converted analogously to β) into CR¹²(OH)R⁵ wherein R¹² is C₁₋₉alkyl and then, if desired, into a compound of formula I wherein R² is C₂₋₁₀alkenyl or C₂₋₁₀alkyl, or the hydroxy group is esterified
and then, if desired, the unsaturated compounds obtained in accordance with α) or β) are reduced to compounds of formula I wherein R² is C₂₋₁₀alkyl,
d) a compound of formula IV 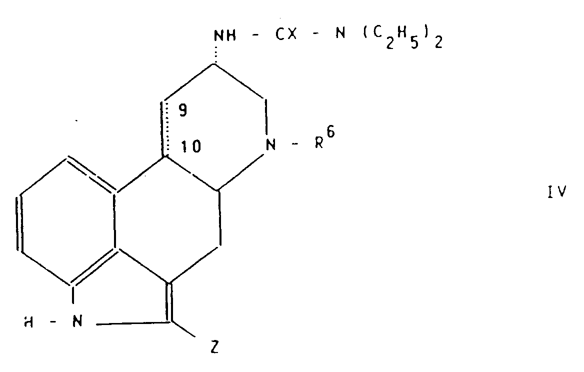 wherein R⁶, X and C₉₋₁₀ are as defined above, and Z is halogen or lithium, is alkylated or alkenylated to a compound of formula I wherein R₂ is C₁₋₁₀alkyl or C₂₋₁₀alkenyl, or
e) a compound of formula V 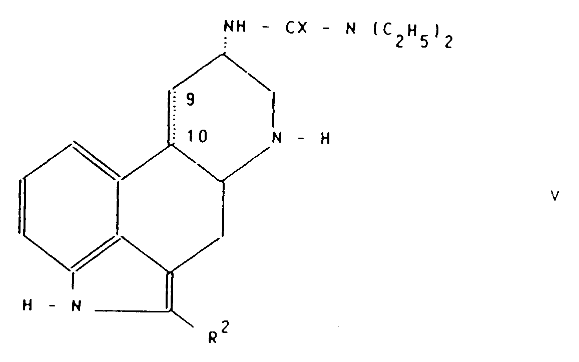 wherein R², X and 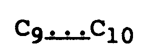 are as defined above, is alkylated in the 6-position
and then, if desired, the compounds of formula I obtained in accordance with processes a-e) wherein X is oxygen are converted into the thioureas or the 2,3-double bond is reduced or the acid addition salts are formed or the isomers are separated.

5. Intermediate compounds of formulae IIa, Ia, Ib and according to claim 4.

## Revendications

1. Composés de formule I 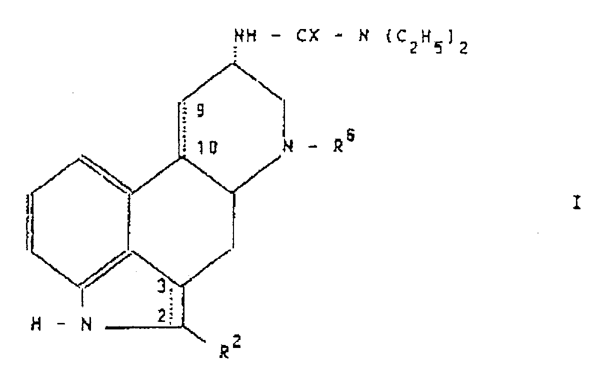 [dans laquelle 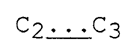 et 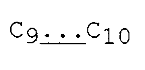 représentent chacun une liaison simple ou une liaison double;
X représente un atome d'oxygène ou de soufre;
R² représente un groupe alkyle en C₁ à C₁₀, un groupe alcényle en C₂ et éventuellement substitué par de l'halogène, un groupe CH₂YR³, CR¹²(OR⁴)R⁵, CH₂-CGR⁹-COR¹⁰ ou COR^{12,}
formules dans lesquelles Y représente un atome d'oxygène ou de soufre; R³ représente un atome d'hydrogène, un groupe alkyle en C₁ à C₄, phényle, acyle en C₂ à C₅ ou phényl-alkyle en C₁ à C₄; R⁴ représente un atome d'hydrogène ou un groupe acyle en C₂ à C₅; R⁵ représente un groupe alkyle en C₁ à C₉; R⁹ représente COCH₃ ou COO-alkyle (en C₁ à C₄); R¹⁰ représente un groupe alkyle en C₁ à C₄ ou O-alkyle en C₁ à C₄; R¹² représente un atome d'hydrogène ou un groupe alkyle en C₁ à C₉; et
R⁶ représente un groupe alkyle en C₂ à C₁₀, alcényle en C₃ à C₁₀ ou cyclo alkyl (en C₃ à C₇)-alkyle (en C₁ à C₃)],
ainsi que leurs sels d'addition d'acides, leurs isomères et mélanges d'isomères.

2. La 3-(6-cyclopropylméthyl-2-méthyl-8α-ergolinyl)-1,1-diéthyl-urée
la 3-(6-allyl-2-méthyl-8α-ergolinyl)-1,1-diéthyl-urée
la 1,1-diéthyl-3-(6-éthyl-2-méthyl-8α-ergolinyl)-1,1-diéthyl-urée
la la 1,1-diéthyl-3-(2-méthyl-6-n-propyl-8α-ergolinyl)-urée
la 1,1-diéthyl-3-(2-méthylthiométhyl-6-n(propyl-8α-ergolinyl)-urée
la 3-(9,10-didéhydro-2-méthylthiométhyl-6-n-propyl-8α-ergolinyl)-1,1-diéthyl-urée
la 3-(6-allyl-2-méthylthiométhyl-8α-ergolinyl)-1,1-diéthyl-urée
la 1,1-diéthyl-3-(6-éthyl-2-méthylthiométhyl-8α-ergolinyl)-urée
la 3-(2-benzyloxyméthyl-6-n-propyl-8α-ergolinyl)-1,1-diéthyl-urée
la 1,1-diéthyl-3-(2-méthoxyméthyl-6-n-propyl-(8α-ergolinyl)-urée
la 3-(9,10-didéhydro-2-méthy-oxyméthyl-6-n-propyl-8α-ergolinyl)-1,1-diéthyl-urée
la 3-(6-allyl-2-méthoxyméthyl-8α-ergolinyl)-1,1-diéthyl-urée
la 1,1-diéthyl-3-(6-éthyl-2-méthoxyméthyl-8α-ergolinyl)-urée
la 1,1-diéthyl-3-(2-hydroxyméthyl-6-n-propyl-8α-ergolinyl)-urée
la 3-(9,10-didéhydro-2-méthyl-6-n-propyl-8α-ergolinyl)-1,1-diéthyl-urée
la 3-(9,10-didéhydro-2-éthyl-6-n-propyl-8α-ergolinyl)-1,1-diéthyl-urée
la 3-(9,10-didéhydro-2-éthyl-2-méthyl-8α-ergolinyl)-1,1-diéthyl-urée
la 1,1-diéthyl-3-(2-méthyl-6-n-propyl)8α-ergolinyl)thiourée
la 1,1-diéthyl-3-(2,3β-dihydro-6 -éthyl-2-méthyl-8α-ergolinyl)-thiourée
la 1,1-diéthyl-3-(2-acétoxyméthyl-6-n-propyl-8α-ergolinyl)-urée
la 1,1-diéthyl-3-[2-(1-hydroxyéthyl-6-n-propyl-8α-ergolinyl] -urée,
la 1,1-diéthyl-3-(éthyl-2-vinyl-8α-ergolinyl)-urée
urée
la 1,1-diéthyl-3-(9,10-didéhydro-6-n-propyl-2-vinyl-8α-ergolinyl)-urée
la 1,1-diéthyl-3-(6-cyclopropylméthyl-2-vinyl-8α-ergolinyl)-urée
la 1,1-diéthyl-3-(6-n-propyl-2-vinyl-8α-ergolinyl)-1,1-diéthyl-urée
la 1,1-diéthyl-3-(2-éthyl-6-n-propyl-8α-ergolinyl)-urée
la 1,1-diéthyl-3-(9,10-didéhydro-2-méthyl-6-n-propyl-8α-ergolinyl)-thiourée
la 1,1-diéthyl-3-(9,10-didéhydro-2,3β-2β-méthyl-6-n-propyl-8α-ergolinyl)-urée
la 1,1-diéthyl-3-(2,3β-dihydro-2-méthyl-6-n-propyl-8α-ergolinyl)-urée,
la 1,1-diéthyl-3-(2,3β-dihydro-2,6-di-n-propyl-8α-ergolinyl)-urée
la (E)-1,1-diéthyl-3-[2-(2-fluoréthylényl)-6-n-propyl-8α-ergolinyl)-urée
la (Z)-1,1-diéthyl-3-[2-(2-fluoréthylényl)-6-n-propyl-8α-ergolinyl)-urée
la 3-(6-allyl-2-éthyl-8α-ergolinyl)-1,1-diéthyl-urée
la 1,1-diéthyl-3-(2,6-diéthyl-8α-ergolinyl)-urée
la 3-(6-cyclopropylméthyl-2-éthyl-8α-ergolinyl)-1,1-diéthyl-urée
la 1,1-diéthyl-3-(2,6-di-n-propyl-8α-ergolinyl)-urée
la 1,1-diéthyl-3-(2-n-hexyl-6-n-propyl-8α-ergolinyl)-urée

3. Médicament à base des composés selon les revendications 1 et 2.

4. Procédé de préparation de composés de formule I selon la revendication 1, selon lequel :
a) on oxyde un composé de formule II 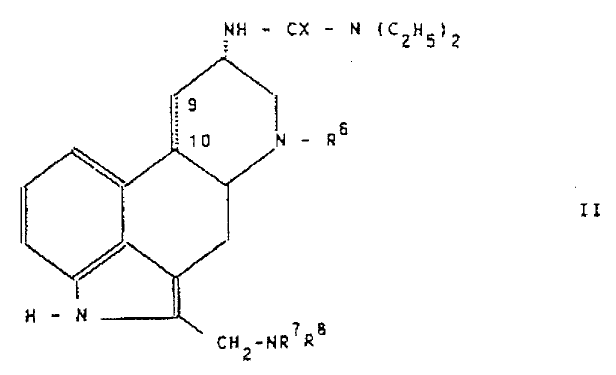 (dans laquelle
R⁶, X et 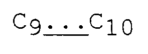 ont le sens ci-dessus, et R⁷ et R⁸ représentent chacun un groupe alkyle en C₁ à C₆ ou forment avec l'atome d'azote un hétérocycle saturé pentagonal à hexagonal, qui peut éventuellement être interrompu par un atome d'oxygène), pour obtenir un aldéhyde de formule Ia 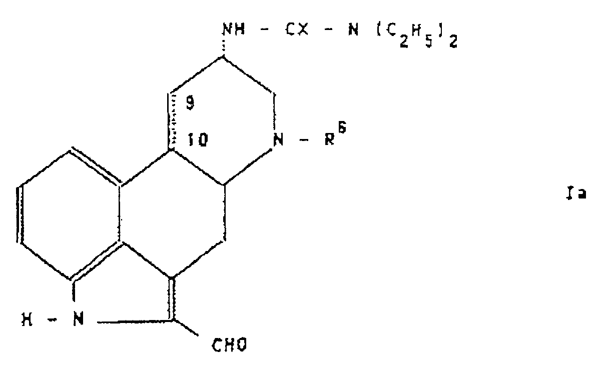 [dans laquelle R₆, X et 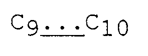 ont le sens ci-dessus];
b) On réduit le sel quaternaire de formule II a 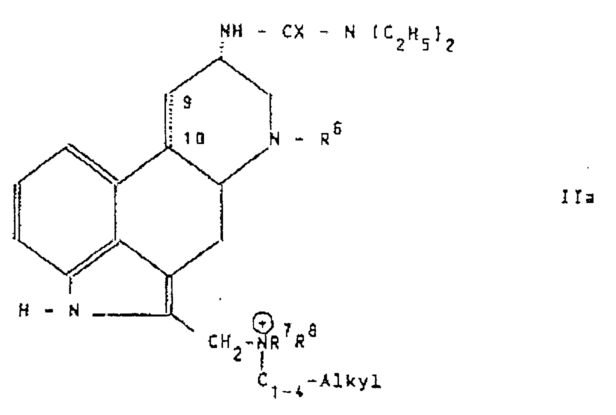 [dans laquelle R⁶, R⁷, R⁸, X et 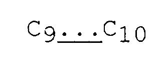 ont le sens ci-dessus] pour obtenir un composé de formule I, (dans laquelle R² représente un groupe méthyle) ou bien on fait réagir avec un anion nucléophile et éventuellement, dans un composé ainsi obtenu de formule I (dans laquelle R² représente un groupe CH₂O benzyle), on scinde le groupe benzyle et éventuellement on estérifie le groupe CH₂OH ainsi obtenu ou bien on l'oxyde pour obtenir un aldéhyde de formule I A,
c) on soumet un composé de formule Ia
α) à une réaction avec un réactif de Wittig, ce qui donne un composé de formule I, dans laquelle R² représente un reste alcényle en C₂-C₁₀ éventuellement substitué par de l'halogène, ou
β) à une transformation en CH(OH)R⁵ et éventuellement ensuite en un composé de formule I (où R² représente un groupe alcényle en C₂ à C₁₀ ou alkyle en C₂ à C₁₀) ou bien on estérifie le groupe hydroxyle ou on l'oxyde pour obtenir une cétone et éventuellement ensuite on transforme le composé ainsi obtenu de formule I b 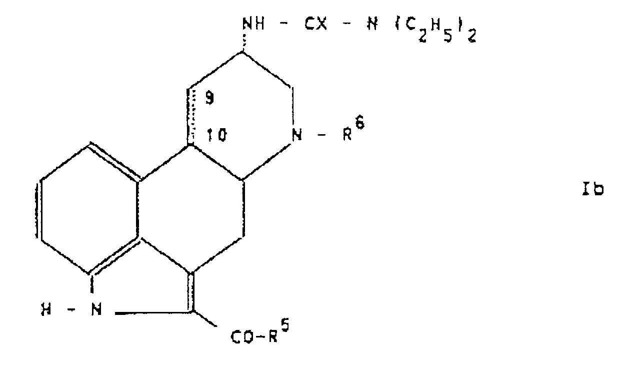
(dans laquelle R⁶, X, R₅ et 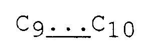 ont le sens précité) de façon analogue à (β) en CR¹² (OH)R⁵ (ou R¹² représente un groupe alkyle en C₁ à C₉) et on transforme éventuellement ensuite en un composé de formule I (dans laquelle R² représente un groupe alcényle en C₂ à C₁₀ ou alkyle en C₂ à C₁₀), ou bien on estérifie le groupe hydroxyle,
et éventuellement ensuite on soumet les composés insaturés, obtenus selon (α) ou selon (β) à une réduction donnant des composés de formule I (dans laquelle R² représente un groupe alkyle en C₂ à C₁₀;
d) on soumet un composé de formule IV 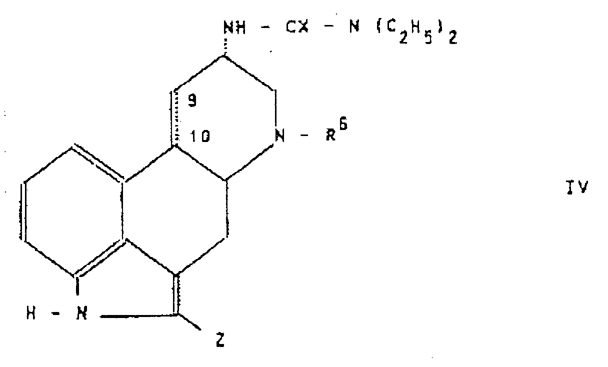 (dans laquelle R⁶, X et C₉-C₁₀ ont le sens indiqué ci-dessus; et z représente un atome d'halogène ou de lithium à une alkylation ou à une alcénylation donnant un composé de formule I (dans laquelle R² représente un groupe alkyle en C₁ à C₁₀ ou alcényle en C₂ à C₁₀),
e) on soumet un composé de formule V 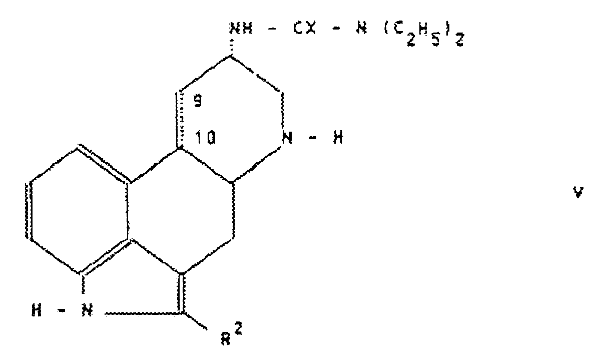 (dans laquelle R², X et 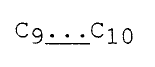 ont le sens précité) à une alkylation en position 6, puis éventuellement on transforme les composés obtenus selon les formes de procédés (a) à (e), de formule I dans laquelle X représente un atome d'oxygène, en les thiourées ou bien on réduit la double liaison en 2-3 ou bien on forme les sels d'addition d'acides ou bien l'on sépare les isomères.

5. Composés intermédiaires, répondant aux formules IIa, 1a, 1b et selon la revendication 4.
